# EUROPEAN PATENT APPLICATION

(11) **EP 2 143 734 A1**
(43) Date of publication of application: **13.01.2010**
(21) Application number: 08715318.5
(22) Date of filing: 25.03.2008
(51) Int. Cl.: C07K 16/18, C07K 7/06, C07K 7/08, C12N 15/09, C12P 21/08, A61K 39/395, A61P 37/02, A61P 29/00, A61P 19/02

(54) **THE FUNCTIONAL EPITOPE OF OSTEOPONTIN, THE ANTI-OSTEOPONIN MONOCLONAL ANTIBODY AND USE THEREOF**

(30) Priority: 24.04.2007 CN 200710039878
(71) Applicant: Shanghai National Engineering Research Center of Antibody Medicine Co., Ltd., Zhangjiang Hi-Tech Park Shanghai 201-203 (CN)
(72) Inventor: GUO, Yajun, Shanghai 201203 (CN); DAI, Jianxin, Shanghai 201203 (CN); WANG, Hao, Shanghai 201203 (CN); KOU, Geng, Shanghai 201203 (CN); HOU, Sheng, Shanghai 201203 (CN); QIAN, Weizhu, Shanghai 201203 (CN); FAN, Kexing, Shanghai 201203 (CN)
(74) Representative: Martin Santos, Victoria Sofia
(86) International application number: PCT/CN2008/070582
(87) International publication number: WO 2008/128456

(57) **Abstract**

The present invention discloses the functional epitope of osteopontin; the anti-osteopontin monoclonal antibody and immune conjugate raising against the functional epitope and their use for preparing anti-tumor drug. The present invention also discloses the DNA sequence encoding the monoclonal antibody, the vector and host cell containing the DNA sequence. The monoclonal antibody and immune conjugate of present invention can also be used to detect OPN. Through inhibiting OPN, the monoclonal antibody or immune conjugate of present invention can remedy or respite autoimmune diseases and protect correlative tissues, so they can be used to remedy autoimmune diseases.

Sequence Listing

<110>  SHANGHAI CP GUOJIAN PHARMACEUTICAL CO.Ltd

<120>  THE FUNCTIONAL EPITOPE OF OSTEOPONTIN, THE
ANTI-OSTEOPONIN MONOCLONAL ANTIBODY AND USE THEREOF

<130>  081705pcwo

<150>  200710039878.6
<151>  2007-04-24

<160>  52

<170>  PatentIn version 3.3

<210>  1
<211>  942
<212>  DNA
<213>  Homo sapiens

<400>  1
atgagaattg cagtgatttg cttttgcctc ctaggcatca cctgtgccat
accagttaaa      60

caggctgatt ctggaagttc tgaggaaaag cagctttaca acaaataccc
agatgctgtg      120

gccacatggc taaaccctga cccatctcag aagcagaatc tcctagcccc
acagaatgct      180

gtgtcctctg aagaaaccaa tgactttaaa caagagaccc ttccaagtaa
gtccaacgaa      240

agccatgacc acatggatga tatggatgat gaagatgatg atgaccatgt
ggacagccag      300

gactccattg actcgaacga ctctgatgat gtagatgaca ctgatgattc
tcaccagtct      360

gatgagtctc accattctga tgaatctgat gaactggtca ctgattttcc
cacggacctg      420

ccagcaaccg aagttttcac tccagttgtc cccacagtag acacatatga
tggccgaggt      480

gatagtgtgg tttatggact gaggtcaaaa tctaagaagt ttcgcagacc
tgacatccag      540

taccctgatg ctacagacga ggacatcacc tcacacatgg aaagcgagga
gttgaatggt      600

gcatacaagg ccatccccgt tgcccaggac ctgaacgcgc cttctgattg
ggacagccgt      660

gggaaggaca gttatgaaac gagtcagctg gatgaccaga gtgctgaaac
ccacagccac     720

aagcagtcca gattatataa gcggaaagcc aatgatgaga gcaatgagca
ttccgatgtg     780

attgatagtc aggaactttc caaagtcagc cgtgaattcc acagccatga
atttcacagc     840

catgaagata tgctggttgt agaccccaaa agtaaggaag aagataaaca
cctgaaattt     900

cgtatttctc atgaattaga tagtgcatct tctgaggtca at
942

<210>  2
<211>  885
<212>  DNA
<213>  Mus musculus

<400>  2
atgagattgg cagtgatttg cttttgcctg tttggcattg cctcctccct
cccggtgaaa      60

gtgactgatt ctggcagctc agaggagaag ctttacagcc tgcacccaga
tcctatagcc     120

acatggccgg tgcctgaccc atctcagaag cagaatctcc ttgcgccaca
gaatgctgtg     180

tcctctgaag aaaaggatga ctttaagcaa gaaactcttc caagcaattc
caatgaaagc     240

catgaccaca tggacgacga tgatgacgat gatgatgacg atggagacca
tgcagagagc     300

gaggattctg tggactcgga tgaatctgac gaatctcacc attcggatga
gtctgatgag     360

accgtcactg ctagtacaca agcagacact ttcactccaa tcgtccctac
agtcgatgtc     420

cccaacggcc gaggtgatag cttggcttat ggactgaggt caaagtctag
gagtttccag     480

gtttctgatg aacagtatcc tgatgccaca gatgaggacc tcacctctca
catgaagagc     540

ggtgagtcta aggagtccct cgatgtcatc cctgttgccc agcttctgag
catgccctct     600

gatcaggaca acaacggaaa gggcagccat gagtcaagtc agctggatga

accaagtctg    660

gaaacacaca gacttgagca ttccaaagag agccaggaga gtgccgatca
gtcggatgtg    720

atcgatagtc aagcaagttc caaagccagc ctggaacatc agagccacaa
gtttcacagc    780

cacaaggaca agctagtcct agaccctaag agtaaggaag atgataggta
tctgaaattc    840

cgaatttctc atgaattaga gagttcatct tctgaggtca actag
885

<210>  3
<211>  348
<212>  DNA
<213>  Artificial

<220>
<221>  misc_feature
<223>  nucleotide sequence of the heavy chain variable region
in 23C3

<400>  3
gaggtgcagc ttgttgagtc tggtggggga ttggtgcagc ctaaagggtc
attgaaatt     60

tcatgtgcag cctctggatt caccttcaat atctacgcca tgaactgggt
ccgccaggct    120

ccaggaaagg gtttggaatg ggttgctcgc ataagaagtc aaagtaataa
ttatacaaca    180

tattatgccg attcagtgaa agacaggttc accatctcca gagatgattc
acaaagcatg    240

ctctatctgc aaatgaacaa cttgaaaact gaggacacag ccatgtatta
ctgtgtgaga    300

caaatgggag actactgggg ccaaggcacc actctcacag tctcctca
348

<210>  4
<211>  116
<212>  PRT
<213>  Artificial

<220>
<221>  misc_feature

<223>  amino acid sequence of the heavy chain variable region
in 23C3

<400>  4

Glu Val Gln Leu Val Glu Ser Gly Gly Gly Leu Val Gln Pro Lys
Gly
1               5                   10                  15

Ser Leu Lys Ile Ser Cys Ala Ala Ser Gly Phe Thr Phe Asn Ile
Tyr
            20                  25                  30

Ala Met Asn Trp Val Arg Gln Ala Pro Gly Lys Gly Leu Glu Trp
Val
        35                  40                  45

Ala Arg Ile Arg Ser Gln Ser Asn Asn Tyr Thr Thr Tyr Tyr Ala
Asp
    50                  55                  60

Ser Val Lys Asp Arg Phe Thr Ile Ser Arg Asp Asp Ser Gln Ser
Met
65                  70                  75                  80

Leu Tyr Leu Gln Met Asn Asn Leu Lys Thr Glu Asp Thr Ala Met
Tyr
                85                  90                  95

Tyr Cys Val Arg Gln Met Gly Asp Tyr Trp Gly Gln Gly Thr Thr
Leu
            100                 105                 110

Thr Val Ser Ser
        115

<210>  5
<211>  324
<212>  DNA
<213>  Artificial

<220>
<221>  misc_feature
<223>  nucleotide sequence of the light chain variable region
in 23C3

<400>  5
tacatccaga tgactcagtc tccagcctcc ctatctgtat ctgtgggaga
aactgtcacc      60

atcacatgtc gagcaagtga gaatatttac agttttttag catggtatca
gcagaaacag     120

ggaaaatctc ctcagctcct ggtctatgct gcaacaaact tagctgatgg
tgtgccatca     180

aggttcagtg gcagtggatc aggcacacag ttttccctca agatcaacag
cctgcagtct     240

gaagattttg ggacttatta ctgtcaacat ttttggggta ctccattcac
gttcggctcg     300

gggacaaagt tggaaataaa acgg
324


<210>  6
<211>  108
<212>  PRT
<213>  Artificial


<220>
<221>  misc_feature
<223>  amino acid sequence of the light chain variable region
in 23C3

<400>  6

Tyr Ile Gln Met Thr Gln Ser Pro Ala Ser Leu Ser Val Ser Val
Gly
1               5                   10                  15


Glu Thr Val Thr Ile Thr Cys Arg Ala Ser Glu Asn Ile Tyr Ser
Phe
            20                  25                  30


Leu Ala Trp Tyr Gln Gln Lys Gln Gly Lys Ser Pro Gln Leu Leu
Val
        35                  40                  45


Tyr Ala Ala Thr Asn Leu Ala Asp Gly Val Pro Ser Arg Phe Ser
Gly
    50                  55                  60

Ser Gly Ser Gly Thr Gln Phe Ser Leu Lys Ile Asn Ser Leu Gln
Ser
65                  70                  75                  80

Glu Asp Phe Gly Thr Tyr Tyr Cys Gln His Phe Trp Gly Thr Pro
Phe
                85                  90                  95

Thr Phe Gly Ser Gly Thr Lys Leu Glu Ile Lys Arg
            100                 105

<210>  7
<211>  348
<212>  DNA
<213>  Artificial

<220>
<221>  misc_feature
<223>  nucleotide sequence of the heavy chain variable region
(VH) in the humanized anti-OPN antibody H23C3

<400>  7
gaggtgcagc tcgttgagag cgggggtggc ctggtccaac ctggagggtc
tcttcgttta     60

tcatgtgcgg ccagcggctt cacttttaac atctacgcca tgaattgggt
ccgccaggca    120

cccggcaagg gacttgagtg ggtggccaga attaggtccc agagtaacaa
ttataccaca    180

tactatgccg attccgttaa agaccggttt acaatcagta gagatgacag
caagaactcc    240

ctctacctgc aaatgaactc tttgaaaacg gaggatactg cagtctatta
ctgcgtaagg    300

cagatgggtg actattgggg ccaaggaacc ctcgtgacag ttagctct
348

<210>  8
<211>  116
<212>  PRT
<213>  Artificial

<220>
<221>  misc_feature
<223>  amino acid sequence of the heavy chain variable region

(VH) in the humanized anti-OPN antibody H23C3

<400> 8

Glu Val Gln Leu Val Glu Ser Gly Gly Gly Leu Val Gln Pro Gly
Gly
1 5 10 15

Ser Leu Arg Leu Ser Cys Ala Ala Ser Gly Phe Thr Phe Asn Ile
Tyr
20 25 30

Ala Met Asn Trp Val Arg Gln Ala Pro Gly Lys Gly Leu Glu Trp
Val
35 40 45

Ala Arg Ile Arg Ser Gln Ser Asn Asn Tyr Thr Thr Tyr Tyr Ala
Asp
50 55 60

Ser Val Lys Asp Arg Phe Thr Ile Ser Arg Asp Asp Ser Lys Asn
Ser
65 70 75 80

Leu Tyr Leu Gln Met Asn Ser Leu Lys Thr Glu Asp Thr Ala Val
Tyr
85 90 95

Tyr Cys Val Arg Gln Met Gly Asp Tyr Trp Gly Gln Gly Thr Leu
Val
100 105 110

Thr Val Ser Ser
        115

<210> 9
<211> 324
<212> DNA
<213> Artificial

<220>
<221> misc_feature
<223> nucleotide sequence of the light chain variable region
(VL) in the humanized anti-OPN antibody H23C3

<400>  9
tacatccaga tgacccagtc cccctcttcc ctgagcgcct ccgtgggcga
ccgcgtgacc     60

atcacctgta gggccagcga gaacatctac tctttcctgg cctggtacca
gcaaaagccc    120

ggcaaggctc cccagctgct cgtgtacgcc gctaccaacc tggccgacgg
cgtgccatcc    180

cgcttcagcg gctccggtag cggcacccag ttcactctga ccatcagctc
cctgcagccc    240

gaggacttcg ccacctacta ttgccagcac ttctggggca ccccttcac
ctttggccag    300

ggtaccaagt tggagatcaa gcgc
324


<210>  10
<211>  108
<212>  PRT
<213>  Artificial


<220>
<221>  misc_feature
<223>  amino acid sequence of the light chain variable region
(VL) in the humanized anti-OPN antibody H23C3

<400>  10

Tyr Ile Gln Met Thr Gln Ser Pro Ser Ser Leu Ser Ala Ser Val
Gly
1               5                   10                  15


Asp Arg Val Thr Ile Thr Cys Arg Ala Ser Glu Asn Ile Tyr Ser
Phe
            20                  25                  30


Leu Ala Trp Tyr Gln Gln Lys Pro Gly Lys Ala Pro Gln Leu Leu
Val
        35                  40                  45


Tyr Ala Ala Thr Asn Leu Ala Asp Gly Val Pro Ser Arg Phe Ser
Gly
    50                  55                  60


Ser Gly Ser Gly Thr Gln Phe Thr Leu Thr Ile Ser Ser Leu Gln

Pro
65                  70                  75                  80

Glu Asp Phe Ala Thr Tyr Tyr Cys Gln His Phe Trp Gly Thr Pro
Phe
                85                  90                  95

Thr Phe Gly Gln Gly Thr Lys Leu Glu Ile Lys Arg
            100                 105

<210>  11
<211>  314
<212>  PRT
<213>  Homo sapiens

<400>  11

Met Arg Ile Ala Val Ile Cys Phe Cys Leu Leu Gly Ile Thr Cys
Ala
1               5                   10                  15

Ile Pro Val Lys Gln Ala Asp Ser Gly Ser Ser Glu Glu Lys Gln
Leu
            20                  25                  30

Tyr Asn Lys Tyr Pro Asp Ala Val Ala Thr Trp Leu Asn Pro Asp
Pro
        35                  40                  45

Ser Gln Lys Gln Asn Leu Leu Ala Pro Gln Asn Ala Val Ser Ser
Glu
    50                  55                  60

Glu Thr Asn Asp Phe Lys Gln Glu Thr Leu Pro Ser Lys Ser Asn
Glu
65                  70                  75                  80

Ser His Asp His Met Asp Asp Met Asp Asp Glu Asp Asp Asp Asp
His
                85                  90                  95

Val Asp Ser Gln Asp Ser Ile Asp Ser Asn Asp Ser Asp Asp Val
Asp
            100                 105                 110

Asp Thr Asp Asp Ser His Gln Ser Asp Glu Ser His His Ser Asp
Glu
        115                 120                 125

Ser Asp Glu Leu Val Thr Asp Phe Pro Thr Asp Leu Pro Ala Thr
Glu
    130                 135                 140

Val Phe Thr Pro Val Val Pro Thr Val Asp Thr Tyr Asp Gly Arg
Gly
145                 150                 155
160

Asp Ser Val Val Tyr Gly Leu Arg Ser Lys Ser Lys Lys Phe Arg
Arg
                165                 170                 175

Pro Asp Ile Gln Tyr Pro Asp Ala Thr Asp Glu Asp Ile Thr Ser
His
            180                 185                 190

Met Glu Ser Glu Glu Leu Asn Gly Ala Tyr Lys Ala Ile Pro Val
Ala
        195                 200                 205

Gln Asp Leu Asn Ala Pro Ser Asp Trp Asp Ser Arg Gly Lys Asp
Ser
    210                 215                 220

Tyr Glu Thr Ser Gln Leu Asp Asp Gln Ser Ala Glu Thr His Ser
His
225                 230                 235
240

Lys Gln Ser Arg Leu Tyr Lys Arg Lys Ala Asn Asp Glu Ser Asn
Glu
                245                 250                 255

His Ser Asp Val Ile Asp Ser Gln Glu Leu Ser Lys Val Ser Arg
Glu
            260                 265                 270

Phe His Ser His Glu Phe His Ser His Glu Asp Met Leu Val Val

Asp
        275                 280                 285

Pro Lys Ser Lys Glu Glu Asp Lys His Leu Lys Phe Arg Ile Ser
His
    290                 295                 300

Glu Leu Asp Ser Ala Ser Ser Glu Val Asn
305                 310

<210>  12
<211>  990
<212>  DNA
<213>  Artificial

<220>
<221>  misc_feature
<223>  nucleotide sequence of the heavy chain constant region
(CH) in the antibody from human

<400>  12
gctagcacca agggcccatc ggtcttcccc ctggcaccct cctccaagag
cacctctggg     60

ggcacagcgg ccctgggctg cctggtcaag gactacttcc ccgaaccggt
gacggtgtcg    120

tggaactcag gcgccctgac cagcggcgtg cacaccttcc cggctgtcct
acagtcctca    180

ggactctact ccctcagcag cgtggtgacc gtgccctcca gcagcttggg
cacccagacc    240

tacatctgca acgtgaatca caagcccagc aacaccaagg tggacaagaa
agttgagccc    300

aaatcttgtg acaaaactca cacatgccca ccgtgcccag cacctgaact
cctgggggga    360

ccgtcagtct tcctcttccc cccaaaaccc aaggacaccc tcatgatctc
ccggacccct    420

gaggtcacat gcgtggtggt ggacgtgagc cacgaagacc ctgaggtcaa
gttcaactgg    480

tacgtggacg gcgtggaggt gcataatgcc aagacaaagc cgcgggaaga
gcagtacaac    540

agcacgtacc gtgtggtcag cgtcctcacc gtcctgcacc aggactggct
gaatggcaag    600

gagtacaagt gcaaggtctc caacaaagcc ctcccagccc ccatcgagaa
aaccatctcc    660

aaagccaaag ggcagccccg agaaccacag gtgtacaccc tgcccccatc
ccgggatgag    720

ctgaccaaga accaggtcag cctgacctgc ctggtcaaag gcttctatcc
cagcgacatc    780

gccgtggagt gggagagcaa tgggcagccg gagaacaact acaagaccac
gcctcccgtg    840

ctggactccg acggctcctt cttcctctac agcaagctca ccgtggacaa
gagcaggtgg    900

cagcagggga acgtcttctc atgctccgtg atgcatgagg ctctgcacaa
ccactacacg    960

cagaagagcc tctccctgtc tccgggtaaa
990


<210>  13
<211>  330
<212>  PRT
<213>  Artificial


<220>
<221>  misc_feature
<223>  amino acid sequence of the heavy chain constant region
(CH) in the antibody from human

<400>  13

<210>  14
<211>  318
<212>  DNA
<213>  Artificial

<220>
<221>  misc_feature
<223>  nucleotide sequence of the light chain constant region
(CL) in the antibody from human

<400>  14
actgtggctg caccatctgt cttcatcttc ccgccatctg atgagcagtt
gaaatctgga     60

actgcctctg ttgtgtgcct gctgaataac ttctatccca gagaggccaa
agtacagtgg    120

aaggtggata acgccctcca atcgggtaac tcccaggaga gtgtcacaga

gcaggacagc    180

aaggacagca cctacagcct cagcagcacc ctgacgctga gcaaagcaga
ctacgagaaa    240

cacaaagtct acgcctgcga agtcacccat cagggcctga gctcgcccgt
cacaaagagc    300

ttcaacaggg gagagtgt
318

<210>  15
<211>  106
<212>  PRT
<213>  Artificial

<220>
<221>  misc_feature
<223>  amino acid sequence of the light chain constant region
(CL) in the antibody from human

<400>  15

Thr Val Ala Ala Pro Ser Val Phe Ile Phe Pro Pro Ser Asp Glu
Gln
1               5                   10                  15

Leu Lys Ser Gly Thr Ala Ser Val Val Cys Leu Leu Asn Asn Phe
Tyr
            20                  25                  30

Pro Arg Glu Ala Lys Val Gln Trp Lys Val Asp Asn Ala Leu Gln
Ser
        35                  40                  45

Gly Asn Ser Gln Glu Ser Val Thr Glu Gln Asp Ser Lys Asp Ser
Thr
    50                  55                  60

Tyr Ser Leu Ser Ser Thr Leu Thr Leu Ser Lys Ala Asp Tyr Glu
Lys
65                  70                  75                  80

His Lys Val Tyr Ala Cys Glu Val Thr His Gln Gly Leu Ser Ser
Pro
                85                  90                  95

Val Thr Lys Ser Phe Asn Arg Gly Glu Cys
            100                 105


<210>  16
<211>  32
<212>  DNA
<213>  Artificial


<220>
<221>  misc_feature
<223>  sense primer for OPN

<400>  16
gggaagctta ccatgagaat tgcagtgatt tg
32


<210>  17
<211>  29
<212>  DNA
<213>  Artificial


<220>
<221>  misc_feature
<223>  anti-sense primer for OPN

<400>  17
gccggtacca ttgacctcag aagatgcac
29


<210>  18
<211>  42
<212>  DNA
<213>  Artificial


<220>
<221>  misc_feature
<223>  sense primer for OPN from mouse

<400>  18
ataagcttgg atgacgacga caagatgaga attgcagtga tt
42


<210>  19
<211>  26
<212>  DNA
<213>  Artificial

<220>
<221>  misc_feature
<223>  anti-sense primer for OPN from mouse

<400>  19
atctcgagtt aattgacctc agaaga
26


<210>  20
<211>  18
<212>  DNA
<213>  Artificial


<220>
<221>  misc_feature
<223>  HGSP1

<400>  20
gatactgtga tctgtttg
18


<210>  21
<211>  18
<212>  DNA
<213>  Artificial


<220>
<221>  misc_feature
<223>  HGSP2

<400>  21
tcgcagatga gtctggac
18


<210>  22
<211>  18
<212>  DNA
<213>  Artificial


<220>
<221>  misc_feature
<223>  HGSP3

<400>  22
atgaacacac tcacattg
18

<210>  23
<211>  24
<212>  DNA
<213>  Artificial

<220>
<221>  misc_feature
<223>  LGSP1

<400>  23
gaggttatga ctttcatagt cagc
24

<210>  24
<211>  24
<212>  DNA
<213>  Artificial

<220>
<221>  misc_feature
<223>  HGSP3

<400>  24
aacactgtcc aggacaccat ctcg
24

<210>  25
<211>  24
<212>  DNA
<213>  Artificial

<220>
<221>  misc_feature
<223>  LGSP1

<400>  25
tctgggatag aagttgttca tgag
24

<210>  26
<211>  21
<212>  DNA
<213>  Artificial

<220>

<221>  misc_feature
<223>  forward primer for GAPDH

<400>  26
cttcaccacc atggagaagg c
21


<210>  27
<211>  21
<212>  DNA
<213>  Artificial


<220>
<221>  misc_feature
<223>  reverse primer for GAPDH

<400>  27
ggcatggact gtggtcatga g
21


<210>  28
<211>  21
<212>  DNA
<213>  Artificial


<220>
<221>  misc_feature
<223>  forward primer for OPN

<400>  28
gatttgcttt tgcctgtttg g
21


<210>  29
<211>  22
<212>  DNA
<213>  Artificial


<220>
<221>  misc_feature
<223>  reverse primer for OPN

<400>  29
tgagctgcca gaatcagtca ct
22


<210>  30

<211>  22
<212>  DNA
<213>  Artificial


<220>
<221>  misc_feature
<223>  forward primer for ¦Áv

<400>  30
ctctgaaacc tgaggtgctt ct
22


<210>  31
<211>  20
<212>  DNA
<213>  Artificial


<220>
<221>  misc_feature
<223>  reverse primer for ¦Áv

<400>  31
gggcgaagta aatgtaacct
20


<210>  32
<211>  21
<212>  DNA
<213>  Artificial


<220>
<221>  misc_feature
<223>  forward primer for ¦Á4

<400>  32
gaatccaaac cagacctgcg a
21


<210>  33
<211>  21
<212>  DNA
<213>  Artificial


<220>
<221>  misc_feature
<223>  reverse primer for ¦Á4

<400>  33
tgacgtagca aatgccagtg g
21


<210>  34
<211>  20
<212>  DNA
<213>  Artificial


<220>
<221>  misc_feature
<223>  forward primer for ¦Á8

<400>  34
ttgaagctgt cagctagacc
20


<210>  35
<211>  20
<212>  DNA
<213>  Artificial


<220>
<221>  misc_feature
<223>  reverse primer for ¦Á8

<400>  35
cacaccacca tcctggtcac
20


<210>  36
<211>  20
<212>  DNA
<213>  Artificial


<220>
<221>  misc_feature
<223>  forward primer for ¦Á9

<400>  36
ttaagtgtcg tgtccatacc
20


<210>  37
<211>  20
<212>  DNA
<213>  Artificial

<220>
<221>  misc_feature
<223>  reverse primer for ¦Á9

<400>  37
gatgttcttc caacgatggg
20


<210>  38
<211>  20
<212>  DNA
<213>  Artificial


<220>
<221>  misc_feature
<223>  forward primer for ¦Â1

<400>  38
cctactggtc ccgacatcat
20


<210>  39
<211>  20
<212>  DNA
<213>  Artificial


<220>
<221>  misc_feature
<223>  reverse primer for ¦Â1

<400>  39
gtcccacttg gcattcattt
20


<210>  40
<211>  22
<212>  DNA
<213>  Artificial


<220>
<221>  misc_feature
<223>  forward primer for ¦Â3

<400>  40
tgatatcctg gtggtactgc tg
22

<210>  41
<211>  22
<212>  DNA
<213>  Artificial

<220>
<221>  misc_feature
<223>  reverse primer for ¦Â3

<400>  41
gttgtttgct gtgtcccact ta
22

<210>  42
<211>  22
<212>  DNA
<213>  Artificial

<220>
<221>  misc_feature
<223>  forward primer for ¦Â5

<400>  42
caacaaatcc tacaatggct ca
22

<210>  43
<211>  22
<212>  DNA
<213>  Artificial

<220>
<221>  misc_feature
<223>  reverse primer for ¦Â5

<400>  43
acaccagagt ctccttgctt tc
22

<210>  44
<211>  19
<212>  DNA
<213>  Artificial

<220>

<221>  misc_feature
<223>  forward primer for CD44

<400>  44
tgaagttggc cctgagcaa
19


<210>  45
<211>  24
<212>  DNA
<213>  Artificial


<220>
<221>  misc_feature
<223>  reverse primer for CD44

<400>  45
ctcggaatta ccacatttcc ttct
24


<210>  46
<211>  23
<212>  DNA
<213>  Artificial


<220>
<221>  misc_feature
<223>  forward primer for IL-1b

<400>  46
caaccaacaa gtgatattct cca
23


<210>  47
<211>  21
<212>  DNA
<213>  Artificial


<220>
<221>  misc_feature
<223>  reverse primer for IL-1b

<400>  47
gatccacact ctccagctgc a
21


<210>  48

<211>  23
<212>  DNA
<213>  Artificial

<220>
<221>  misc_feature
<223>  forward primer for IL-6

<400>  48
gaggatacca ctcccaacag acc
23

<210>  49
<211>  24
<212>  DNA
<213>  Artificial

<220>
<221>  misc_feature
<223>  reverse primer for IL-6

<400>  49
aagtgcatca tcgttgttca taca
24

<210>  50
<211>  23
<212>  DNA
<213>  Artificial

<220>
<221>  misc_feature
<223>  forward primer for TNF-¦Á

<400>  50
catcttctca aaattcgagt gac
23

<210>  51
<211>  22
<212>  DNA
<213>  Artificial

<220>
<221>  misc_feature
<223>  reverse primer for TNF-¦Á

<400>  51
tgggagtaga caaggtacaa cc
22

<210>  52
<211>  66
<212>  DNA
<213>  Artificial

<220>
<221>  misc_feature
<223>  sequence of the signal peptide gene

<400>  52
atggattttc aggtgcagat tttcagcttc ctgctaatca gtgcctcagt
cataatatcc     60

agagga
66

## Description

### Technical field

The present invention relates to immunology field. In particular, the present invention discloses a functional protein epitope, monoclonal antibodies specially binding to the same, and the use of these antibodies.

### Technical Background

Autoimmune refers to the phenomenon that the organism immune system produces antibodies and sensitized lymphocytes against its own tissue or organ, and induces immune response. When autoimmune causes functional dysfunctions of the tissue or organ and results in clinical symptoms, it is termed as autoimmune disease (AID). More than 30 autoimmune diseases are currently known, most of which are primary diseases and only a few of which are secondary diseases. Primary autoimmune disease, which is caused by unknown causes, is closely related to genetic factors and can be divided into organ specific and non-organ specific. The target antigens and the lesions of organ-specific AID are often restricted to a particular organ, while the target antigens and the lesions of non-organ specific AID often appear as systemic or systematic. In recent years, autoimmune and autoimmune diseases are of more and more attention. About several hundreds millions of people are suffering from the pain caused by the disease throughout the world. The disease has a longer duration, can cause disability, and is even deadly when serious. Due to the universality and seriousness of this problem, a global research wave of autoimmune disease is set off.

The pathogenesis of autoimmune diseases is very complex, and the exact pathogenesis is currently not fully understood. For the time being, it is believed that, individuals with certain genetic characteristics (determined by AID-related gene polymorphisms) may suffer from autoimmune disease for some internal and external pathogenic factors stimuli (autoantigen release, allosteric and ultra-antigen molecular simulation, etc.) or gene mutation, modification of autoreactive immune cells, which result in antigen-presenting cell (APC) or dendritic cell abnormal activation, immune regulatory network imbalance (TH1/TH2 balance destruction) and changes in autoreactive immune cells polyclonal activation or apoptosis inhibition. The onsets of autoimmune diseases related to many factors, including genetic, infection, immune dysfunction and so on. Although the pathogenesis is still not clear, but a large number of studies have indicated that the levels of a variety of inflammatory factors, such as TNF-a, IL-1β and IL-6 and so on in autoimmune disease patients are much higher than the health individual. Furthermore, anti-TNF-a monoclonal antibody and IL-1 receptor antagonist have showed significant therapeutic effect, which greatly improved the patient's condition, especially for some autoimmune diseases, such as rheumatoid arthritis, ankylosing spondylitis, Crohn's disease, etc. ("The therapeutic effects of an engineered human anti-tumour necrosis factor alpha antibody (CDP571) in rheumatoid arthritis". 1995. Br. J. Rheumatol. 34: 334-342; "Infliximab (chimeric anti-tumour necrosis factor alpha monoclonal antibody) versus placebo in rheumatoid arthritis patients receiving concomitant methotrexate: a randomised phase III trial". ATTRACT Study Group. Lancet. 1999. 354: 1932-1939; "Infliximab and methotrexate in the treatment of rheumatoid arthritis". N. Engl. J. Med. 2000. 343: 1594-1602). Although these biological treatments have produced significant therapeutical effects, they still can not completely alleviate and cure these diseases. In recent years, a large number of studies indicated that chemokines also play an important role in autoimmune diseases, they can accurately regulate and control the migration of leukocytes to the inflammatory tissue, and reduce the apoptotic activity, extend the life of B-cell and T-cell, so as to attack the target tissue.

Osteopontin (OPN), also known as early T-lymphocyte-activation factor I (Eta-1) or secreted phosphoprotein I, contains Arg-Gly-Asp (RGD, arginine-glycine- aspartic acid) tripeptide sequence, has a molecular weight of about 44-66kD depending on the dephosphorylation and glycosylation forms, and is an important multifunctional extracellular matrix protein ("Eta-1 (osteopontin): an early component of type-1 (cell-mediated) immunity". Science. 2000. 287: 860-864). OPN is expressed in bone tissue cells, macrophages, activated T cells and epithelial cells, etc., and plays an important role in mediating cell chemotaxis, adhesion, proliferation and migration, tumor metastasis, bone mineralization and reconstruction, immune regulation, signal transduction, as well as immunity to infectious diseases, etc. ("Osteoprotegerin Ligand Is a Cytokine that Regulates Osteoclast Differentiation and Activation". Cell, 1998. 93: 65-176. "Osteopontin: role in cell signaling and cancer progression". TRENDS in Cell Biology 2006.16: 79-87.). OPN binds to cell surface receptors mainly through RGD pathway and non-RGD pathway so as to achieve its function ("Inhibition of Arg-Gly-Asp (RGD) -mediated Cell Adhesion to osteopontin by a Monoclonal Antibody against osteopontin". J. Bio. Chem. 1994. 269: 23280-23285), wherein in the RGD pathway, OPN mainly binds to cell surface integrin receptors (αvβ3, αvβ1, αvβ5), and in non-RGD pathway, it mainly binds to some variants of CD44 (V6, V7), thereby mediates a variety of cell physiological functions ("Intracellular Osteopontin Is an Integral Component of the CD44-ERM Complex Involved in Cell Migration". Journal of Cellular Physiology. 2000. 184: 118-130). In general, OPN secreted by cells exists in the body in many forms after a series of post-expression modifications such as phosphorylation, glycosylation, and cleavage by thrombin, and every form may have its unique function which is not fully explained based on the current study and needs further research and discussion.

A large number of studies indicate that in autoimmune diseases (rheumatoid arthritis, multiple sclerosis, autoimmune hepatitis, Crohn's disease, etc.), the tissue and plasma OPN levels were significantly up-regulated ("Expression of osteopontin at sites of bone erosion in a murine experimental arthritis model of collagen-induced arthritis: possible involvement of osteopontin in bone destruction in arthritis". Arthritis Rheum. 2002. 46: 1094-1101; "Role of osteopontin in amplification and perpetuation of rheumatoid synovitis". J. Clin. Invest. 2005. 115: 1060-1067. "Osteopontin/Eta-1 upregulated in Crohn's disease regulates the Th1 immune response". Gut. 2005. 54: 1254-1262. "Osteopontin as a Mediator of NKT Cell Function in T Cell-Mediated Liver Diseases". Immunity, 2004. 21: 539-550). OPN recruits inflammatory cells in the target organ by binding to CD44 and other integrin receptors on the surface of lymphocytes and macrophages, and further promotes the activation of various inflammatory cells, which secretes a large number of pro-inflammatory cytokines (TNF-α, IL-1β and IL-6) ("Essential role of the cryptic epitope SLAYGLR within osteopontin in a murine model of rheumatoid arthritis". J. Clin. Invest. 2003. 112: 181-188). In addition, in rheumatoid arthritis, OPN can mediate bone destruction and absorption through binding to αvβ3 and αvβ5 receptors on the surface of osteoclast, thereby increase the onset of RA ("Osteopontin". Crit Rev Oral Biol Med. 2000; 11(3): 279-303). Studies have shown that, in OPN gene deletion mice, the incidence and severity of arthritis decreased significantly ("Osteopontin deficiency protects joints against destruction in anti-type II collagen antibody induced arthritis in mice". PNAS. 2002. 99(7): 4556-4561), the incidence of ConA-induced autoimmune hepatitis and hepatic injury level also decreased significantly ("Osteopontin as a Mediator of NKT Cell Function in T Cell-Mediated Liver Diseases". Immunity, 2004. 21: 539-550), and a protective effect on DSS-induced Crohn's disease in mice was also observed ("Osteopontin deficiency protects mice from Dextran sodium sulfate-induced colitis". Inflamm Bowel Dis. 2006. 12(8): 790-6).

In the above models, the anti-OPN polyclonal antibody can significantly lower the incidence in rheumatoid arthritis model mice, reduce the extent of joint swelling and deformity ("Essential role of the cryptic epitope SLAYGLR within osteopontin in a murine model of rheumatoid arthritis". J. Clin. Invest. 2003. 112: 181-188), and can also significantly reduce the liver inflammation ("Osteopontin as a Mediator of NKT Cell Function in T Cell-Mediated Liver Diseases". Immunity, 2004. 21: 539-550). Thus, OPN is a major pathogenic factor of autoimmune diseases and will be an effective drug target for the treatment of autoimmune diseases.

### Description of the Invention

The purpose of this invention is to provide a monoclonal antibody against the therapeutic target, osteopontin, for the treatment of autoimmune diseases.

The first aspect of the present invention provides a functional osteopontin epitope,
wherein the functional epitope is WLXPDP, and wherein X is any amino acid.

In a preferred embodiment, the preferred X in the functional epitope is: N, M or L.

The second aspect of the present invention provides an anti-osteopontin monoclonal antibody which specifically binds to the above functional epitope.

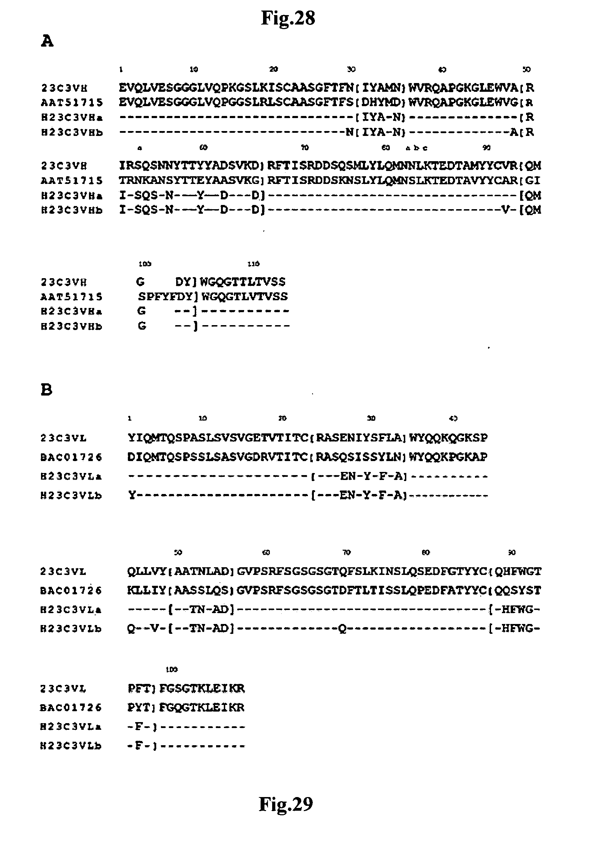

In one embodiment, the amino acid sequence of the CDR in the VH of the monoclonal antibody is selected from the group consisting of: IYAMD, RIRSQSNNYTTYYADSVKD and QMGDY (See, for example, H23C3VHb in Figure 29); the amino acid sequence of the CDR in the VL is selected from the group consisting of: RASENIYSFLA, AATNLAD and QHFWGTPFT (See, for example, H23C3VLb in Figure 29).

In another embodiment, the amino acid sequence of the VH in the monoclonal antibody is as set forth in SEQ ID NO: 4 or SEQ ID NO: 8, and the amino acid sequence of the VL is as set forth in SEQ ID NO: 6 or SEQ ID NO: 10.

In another embodiment, the constant region of the monoclonal antibody is a mouse constant region or a human constant region.

In a preferred embodiment, the constant region is a human constant region. In another preferred embodiment, the amino acid sequence of the heavy chain constant region in the constant region is as set forth in SEQ ID NO: 13. In another preferred embodiment, the amino acid sequence of the light chain constant region in the constant region is as set forth in SEQ ID NO: 15. In another preferred embodiment, the monoclonal antibody is obtained by hybridoma method or recombinant DNA method, or is isolated from phage antibody library. In another preferred embodiment, the monoclonal antibody is a chimeric antibody or a humanized antibody.

The third aspect of the present invention provides a DNA molecule, which encodes the monoclonal antibody of this invention as mentioned above.

In another preferred embodiment, the VH encoding nucleotide sequence in the DNA molecule is as set forth in SEQ ID NO: 3 or SEQ ID NO: 7; and the VL encoding nucleotide sequence is as set forth in SEQ ID NO: 5 or SEQ ID NO: 9.

The fourth aspect of the present invention provides a vector, which contains the DNA molecule of the present invention as mentioned above.

The fifth aspect of the present invention provides a host cell, which contains the vector of the present invention, or has the DNA molecule of the present invention integrated in its genome.

In a preferred embodiment, the host cell is a prokaryotic cell, preferably a bacterial cell; a lower eukaryotic cell, preferably a yeast cell; or a higher eukaryotic cell, preferably a mammalian cell.

The sixth aspect of the present invention provides an immunoconjugate, which contains: (a) the monoclonal antibody of the present invention; and (b) a conjugate moiety selected from the group consisting of drugs, toxins, cytokines, radionuclides, and enzymes.

The seventh aspect of the present invention provides a use of the anti-osteopontin monoclonal antibody or immunoconjugate of the present application in the manufacture of a medicament for treating autoimmune diseases.

In another preferred embodiment, the autoimmune disease is selected from the group consisting of rheumatoid arthritis, multiple sclerosis, autoimmune hepatitis, ankylosing spondylitis, Crohn's disease, rheumatoid spondylitis, osteoarthritis, gouty arthritis, autoimmune diabetes, autoimmune uveitis, nephrotic syndrome, ulcerative colitis, psoriatic arthritis, undifferentiated spondyloarthropathy, chronic liver failure, and other rheumatoid arthritis-related conditions including weight gain, joint distortion, joint swelling, joint deformity, joint bending stiffness, severe movement disorders, and combinations thereof.

The eighth aspect of the present invention provides a pharmaceutical composition comprising the anti-osteopontin monoclonal antibody or immunoconjugate of the invention and the pharmaceutically acceptable carriers.

In a preferred embodiment, the pharmaceutical composition contains 0.00001 - 99.9%, preferably 0.0001-90 wt%, more preferably 0.001-75 wt%, even more preferably 0.01-50 wt% of the monoclonal antibody or immunoconjugate. In another preferred embodiment, the pharmaceutical composition further comprises other active agents selected from the group consisting of anti-inflammatory cytokines including IL-4, IL-6, IL-10 and/or IL-4 antagonist; ENBREL®; phosphodiesterase IV inhibitors; anti-inflammatory inhibitors and cytokine inhibitors such as leflunomide; non-steroidal inflammatory drugs such as naproxen, cortisol, or cyclosporine.

The ninth aspect of the present invention provides a kit for detection of osteopontin, which contains the anti-osteopontin monoclonal antibody or immunoconjugate of the invention.

The tenth aspect of the present invention provides a method for the detection of the presence or the content of osteopontin in a biological sample, which includes the following steps: (i) contacting the sample with the anti-osteopontin monoclonal antibody or immunoconjugate of the invention; (ii) detecting the formation of antigen - antibody complex, wherein the formation of antigen - antibody complex suggests the presence of osteopontin in the sample, or the quantitative determined content of the antigen - antibody complex reflects the content of osteopontin in the sample.

In a preferred embodiment, the sample can be optionally pre-treated, preferably pretreated by extraction, purification, or enrichment.

Other aspects of the present invention will be obvious to one skilled in the art from the disclosure herein.

### DESCRIPTION OF THE DRAWINGS

Figure 1: SDS-PAGE electrophoretogram of human and mouse OPN after eukaryotic expression and purification, wherein M represents protein molecular weight marker.
Figure 2: Western blot using 23C3 as primary antibody.
Figure 3: Effect of 23C3 mAb treatment on clinical scores, onset time and incidence rate for arthritis in CIA mice. Figure 3a is the clinical scores of arthritis of CIA mice in different treatment groups, Figure 3b is the photographs of the arthritic joint of CIA mice in different treatment groups, and Figure 3c is the incidence of CIA mice in different treatment groups.
Figure 4: The protective effect of 23C3 mAb treatment on joint bone resorption in CIA mice.
Figure 5: Effect of 23C3 mAb on *in vitro* inhibition of lymphocyte migration.
Figure 6: OPN level in the plasma and joint tissue of CIA mice in different treatment groups; Figure 6a is the plasma OPN level, and Figure 6b is OPN mRNA level in the joint tissue.
Figure 7: The mRNA Levels of OPN receptors in the joint tissue of CIA mice in different treatment groups.
Figure 8: The mRNA Levels of various cytokines in the joint tissue of CIA mice in different treatment groups.
Figure 9: *In vitro* lymphocyte recall response to collagen type II in different treatment groups; Figure 9a is the result of *in vitro* lymphocyte recall response in different treatment groups, and Figure 9b is the result of cytokines levels in the *in vitro* lymphocyte recall response of different treatment groups.
Figure 10: Serum autoantibody levels of CIA mice in different treatment groups.
Figure 11: RANKL/RANK/OPG mRNA levels in the joint tissue of CIA mice in different treatment groups.
Figure 12: Urine D-pyr level of CIA mice in different treatment groups.
Figure 13: Effect of 23C3 mAb on mortality in mouse model for hepatitis.
Figure 14: Effect of 23C3 mAb on liver injury in mouse model. Figure 14a is the plasma ALT and AST levels in different treatment groups; and Figure 14b is the result of liver HE and TUNEL staining in different treatment groups. Figs A, C, E: HE staining; Figs B, D, F: TUNEL staining.
Figure 15: Plasma and liver tissue OPN levels in model mice for hepatitis of different treatment groups.
Figure 16: Plasma and liver tissue cytokine levels in model mice for hepatitis of different treatment groups. Figure 16a is the result of serum TNF-α, IFN-g levels in different treatment groups, and Figure 16b is the result of mRNA levels of various cytokines in liver tissue of different treatment groups.
Figure 17: Effect of 23C3 mAb on body weight, disease activity index (DAI), and spleen index in mouse model for Crohn's disease. Figure 17a is the change of mice body weight in different treatment groups, Figure 17b is the disease activity index (DAI) in different treatment groups, and Figure 17c is the spleen index in different treatment groups.
Figure 18: Histopathology of intestinal tissue in mouse model for Crohn's disease of different treatment groups.
Figure 19: Effect of 23C3 mAb on myeloperoxidase (MPO) activity of intestinal tissue in mouse model for Crohn's disease.
Figure 20: TNF-α, IL-1β and IL-6 mRNA levels in the intestinal tissue of mouse model for Crohn's disease in different treatment groups.
Figure 21: OPN level in the intestinal tissue and plasma of mouse model for Crohn's disease in different treatment groups. The left panel is the result of plasma OPN level, and the right panel is the result of OPN level in intestinal tissue.
Figure 22: The comparison of output effects after three cycles of 23C3 panning.
Figure 23: ELISA and Western blot of 23C3-positive phage; Figure 23A is the ELISA result of 23C3-positive phage, and Figure 23B is the Western blot result of 23C3-positive phage. In Figure 23B, the left panel is the hybridization result of antibody 23C3, and the right panel is the hybridization result of irrelevant antibody SM5-1.
Figure 24: Align X sequence analysis of 23C3 binding epitope.
Figure 25: Comparison of the binding ability between a variety of sequence epitopes and antibody 23C3.
Figure 26: The specific binding of synthetic short peptide to corresponding antibody and the blocking of antibody -antigen binding; Figure 26A is the dot blot result of the binding between short peptide and antibody, and Figure 26B is the result of short peptide blocking.
Figure 27: The relative position of 23C3-specific epitope on the OPN molecule.
Figure 28: The schematic diagram of humanized antibody h23C3 molecular modeling structure; FR residues are represented in dark gray stripes, CDR residues are represented in light gray stripes, and the seven FR residues within 5Å spatial area around CDR that may affect the conformation of original antibody CDR but have different positions to the corresponding residues in human source template are in black balls and rods.
Figure 29: The alignment between the amino acid sequences of the heavy chain (Figure 29A) and the light chain (Figure 29B) in humanized antibody h23C3 and relevant sequences, wherein 23C3VH and 23C3VL represent variable regions in the heavy chain and the light chain of monoclonal antibody 23C3 from mouse, respectively; VH of human antibody AAT511715.1 and VL of human antibody BAC01726.1 are selected to construct the FRs in the heavy chain and the light chain of the humanized antibody h23C3, respectively; H23C3VHa and H23C3VHb represent the VHs of different humanized antibodies, respectively; h23C3VLa and h23C3VLb represent the VLs of different humanized antibodies, respectively; dash represents the same amino acids as the corresponding residue of human antibody AAT51715.1 or BAC01726.1; CDR is shown in the parentheses; and amino acids are numbered according to Kabat numbering method [E.A. Kabat, T.T. Wu, H.M. Perry, K.S. Gottesman, C. Foeller, "Sequences of Proteins of Immunological Interest", 5th edition, United States Department of Health and Human Services, Bethesda, MD, 1991.].
Figure 30: Antigen-binding activity experiment using 23C3 humanized antibody.
Figure 31: Western blot using c23C3 or h23C3 as primary antibody.
Figure 32: Effect of c23C3 and h23C3 antibody on the lymphocyte migration.
Figure 33: Effect of c23C3 and h23C3 antibody on the lymphocyte immune response.
Figure 34: Effect of c23C3 and h23C3 antibody on the survival of activated lymphocytes.

### DESCRIPTION OF THE EMBODIMENTS

The inventors obtained the monoclonal antibody against specific functional epitope of osteopontin after long and thorough studies, further produced chimeric monoclonal antibody and humanized antibody, and determined the encoding sequences thereof. By studying, the inventors also demonstrated that the monoclonal antibody of the present invention can relieve or alleviate the symptoms of, for example, arthritis, autoimmune hepatitis, Crohn's disease and other autoimmune diseases by inhibiting osteopontin, protect related organs, and thus can be used for the treatment of autoimmune diseases. On the basis of these findings, the present invention was accomplished.

In particular, the applicant of the present invention cloned human and mouse OPN genes using molecular biology technology, expressed human and mouse OPN proteins in eukaryotic cells, and prepared anti-human OPN monoclonal antibody (for example, 23C3 of the present invention) by cell fusion - hybridoma method, as well as cloned and sequenced the obtained monoclonal antibody gene.

The invention further disclose a method for the preparation of a humanized anti-osteopontin antibody, including the steps of computer-aided designing of humanized antibody h23C3 amino acid sequence; whole gene synthesizing h23C3 heavy chain and VL genes and splicing the same with human antibody heavy and light chain constant regions by gene recombination; cloning the obtained recombinant into an eukaryotic expression vector to construct humanized antibody light and heavy chain expression vectors respectively; co-transfecting a host cell (eg. CHO) with said light and heavy chain expression vectors by liposome transfection method; screening and culturing the vectors; and obtaining the antibody by purification. Chimeric monoclonal antibody c23C3 can also be obtained according to this method.

The applicant of the present invention has also established a collagen-induced arthritis DBA/1J mouse model, ConA-induced autoimmune hepatitis model, and DSS-induced Crohn's disease mouse model. Experiments were carried out in the three autoimmune disease models with the above monoclonal antibodies to determine the protective effects.

The experimental results of arthritis model showed that, monoclonal antibody of the present invention could significantly reduce the clinical scores of arthritis, delay onset time, inhibit the function of osteoclasts, reduce the bone destruction and absorption caused by arthritis, as well as protect bone resorption, effectively block lymphocyte migration, and so as to reduce or alleviate arthritis symptoms.

The experimental results of autoimmune hepatitis model showed that the monoclonal antibodies could neutralize OPN protein in liver tissue, regulate OPN mRNA level, reduce the level of pro-inflammatory cytokines, down-regulate NF-κB level in Th1-type immune response, so as to achieve the purpose of protecting liver and reducing mortality.

The result of experiments using Crohn's disease model showed that the monoclonal antibodies could neutralize OPN protein in liver tissue, regulate OPN mRNA level, decrease MPO activity, reduce lymphocyte and neutrophil infiltration, reduce the level of pro-inflammatory cytokines, thereby reducing inflammation and ulceration, protecting intestinal and/or colon tissue, alleviating the symptoms of Crohn's disease, so as to achieve the prevention and/or therapeutic purposes.

The applicant of the present invention has also identified the OPN functional epitope interacting with 23C3 mAb by phage display technology, wherein the functional epitope is WLXPDP, and X can be any amino acids, more preferably X in the functional epitope is N, M or L. Therefore, the target of OPN molecular was further disclosed. More specifically, the applicant of the present invention has inferred different OPN functional epitopes by monoclonal antibody epitope panning, phage ELISA and Western blot, sequencing and sequence analysis, and further synthesized a series of short peptides by analyzing phage and antibody binding ability and choosing the clone with the strongest binding ability. The functional epitopes were identified by short peptide-specific antibody binding assay.

### Monoclonal antibody of the invention and the preparation thereof

As used herein, the term "monoclonal antibody (mAb)" refers to antibodies from a substantially homogeneous population, that is, the individual antibodies in the population are substantially the same, except for some naturally occurring mutations. A monoclonal antibody is highly specific to a unique antigenic site. Moreover, unlike conventional polyclonal antibody formulations (which are usually different antibodies specific to different epitopes), each monoclonal antibody is specific to a unique epitope on an antigen. In addition to the specificity, the advantages of monoclonal antibodies also include that, they can be synthesized by a hybridoma culture and will not be contaminated by other immunoglobulins. The modifier "monoclonal" indicates the characteristics of the antibody, which is from a substantially homogeneous antibody population, and should not be interpreted as being limited to antibodies produced by any special method.

As used herein, the term "antibody" or "immunoglobulin" refers to a heterotetramerized glycoprotein of about 150,000 Dalton and having the same structural characteristics, which consists of two identical light chains (L) and two identical heavy chains (H). Each light chain is linked to the heavy chain through a covalent disulfide bond, and different immunoglobulin isotypes have different numbers of disulfide bonds between the two heavy chains. Each heavy chain and each light chain also has regularly spaced interchain disulfide bonds. One end of each heavy chain is a variable region (VH), followed by multiple constant regions. Each light chain has a variable region (VL) at one end, and a constant region at the other end. The light chain constant region is located opposite to the first heavy chain constant region, and the light chain variable region is opposite to the heavy chain variable region. Specific amino acid residues form an interface between the light chain and the heavy chain variable regions.

As used herein, the term "variable" refers to some parts of the antibody variable regions, which have difference sequences and contribute to the binding specificity between specific antibodies to their specific antigens. However, variability is not evenly distributed throughout the variable regions of antibody. It is concentrated in three fragments in the light chain and heavy chain variable regions, which are known as complementarity determining region (CDR) or hypervariable region. The relatively conservative part of the variable regions is known as the framework region (FR). Each of the naturally occurred heavy chain and light chain variable regions independently contains four FRs, which are generally in β-folding configuration, connected with three CDRs forming connection loops, and in some cases can form part of the β-folding structure. The CDRs in each chain are closely positioned through FR and form the antigen binding site of the antibody together with the CDRs of another chain (see Kabat, etc., NIH Publ. No. 91-3242, Volume I, p647-669, (1991)). The constant regions are not directly involved in the binding of antibody and antigen, but show different effects, such as involved in antibody-dependent cytotoxicity of an antibody.

The "light chains" of a vertebrate antibody (immuneglobulin) may be classified as one of two significantly different classes (known as κ and λ) based on the amino acid sequences of its constant region. In accordance with the amino acid sequences of CH, immunoglobulins can be divided into different types. There are five types of immunoglobulins: IgA, IgD, IgE, IgG and IgM, some of which can be further divided into subtypes (isotype), such as IgG1, IgG2, IgG3, IgG4, IgA, and IgA2. The heavy chain constant region corresponding to different types of immunoglobulin, are known as α, δ, ε, γ, and µ. The subunit structures and three-dimensional conformations of different types of immunoglobulins are well known in the art.

For example, in the present invention, the amino acid sequence of the VH in the monoclonal antibody is preferably as set forth in SEQ ID NO: 4 or SEQ ID NO: 8, the amino acid sequence of the VL is preferably as set forth in SEQ ID NO: 6 or SEQ ID NO: 10, while the constant region can be from mouse or human, such as IgG from mouse.

The monoclonal antibody obtained by the present molecular biology techniques also comprise the chimeric monoclonal antibody c23C3 and humanized antibody h23C3 which specific bind to the above osteopontin functional epitope. In c23C3, the amino acid sequence of the VH is as set forth in SEQ ID NO: 4, the amino acid sequence of the VL is as set forth in SEQ ID NO: 6, and the constant region is from human antibody. In h23C3, the amino acid sequence of the VH is as set forth in SEQ ID NO: 8, the amino acid sequence of the VL is as set forth in SEQ ID NO: 10, and the constant region is from human antibody.

In one embodiment of the present invention, the CDR1-3 amino acid sequences of the VH are independently selected from the group consisting of IYAMD, RIRSQSNNYTTYYADSVKD and QMGDY (see H23C3VHb in Figure 29; and the CDR1-3 amino acid sequences of the VL are independently selected from the group consisting of RASENIYSFLA, AATNLAD and QHFWGTPFT(see H23C3VLb in Figure 29).

The monoclonal antibodies can be prepared using a variety of well known methods in the art. For example, the monoclonal antibodies can be prepared by hybridoma method (first proposed by Kohler et al., Nature, 256:495 (1975)), or recombinant DNA method (U.S. Patent No. 4,816,567). The monoclonal antibodies can also be isolated from phage antibody library according to the techniques as disclosed for example, in Clackson et al., Nature, 352:624-628 (1991) and Marks et al., J. Mol. Biol., 222:581-597 (1991).

The monoclonal antibody of the present invention can also be a chimeric antibody or a humanized antibody, such as the chimeric monoclonal antibody c23C3 and the humanized antibody h23C3 obtained by known molecular biology techniques. In c23C3, the amino acid sequence of the VH is as set forth in SEQ ID NO: 4, the amino acid sequence of VL is as set forth in SEQ ID NO: 6, and the constant region is from human antibody. In h23C3, the amino acid sequence of the VH is as set forth in SEQ ID NO: 8, the amino acid sequence of the VL is as set forth in SEQ ID NO: 10, and the constant region is from human antibody.

The invention also includes the monoclonal antibody having corresponding amino acid sequence of the anti-hOPN monoclonal antibody, the monoclonal antibody having the variable region chain(s) of the anti-hOPN monoclonal antibody, and other proteins or protein conjugates as well as products of fusion expression having these chains. Specifically, the present invention includes any proteins or protein conjugates as well as any product of fusion expression (i.e. immunoconjugate and fusion expression products) having hypervariable region (complementary determining region, CDR) in the light chain and the heavy chain thereof, as long as the hypervariable regions in the light chain and the heavy chain are identical or have at least 90% homology, preferably at least 95% homology, to the variable regions in the light chain and the heavy chain of the present invention. As known to the skilled in the art, the immunoconjugate and the products of fusion expression include the conjugates formed by binding drugs, toxins, cytokines, radionuclides, enzymes and other diagnostic or therapeutic molecules with the anti-hOPN monoclonal antibody or the fragments thereof. The invention also includes cell surface markers or antigens binding to the anti-hOPN monoclonal antibody or the fragments thereof.

The invention includes not only the complete monoclonal antibody, but also immuno-active antibody fragments, such as Fab or (Fab')₂ fragments; a heavy chain of the antibody; and a light chain of the antibody.

### Molecules encoding anti-hOPN monoclonal antibody or its fragments, expression vectors and host cells containing the molecules

The present invention also provides DNA molecules that encode the anti-hOPN monoclonal antibody or the fragments thereof. The sequences of these DNA molecules can be obtained by conventional techniques, such as PCR amplification or genome library screen. In addition, a sequence encoding the light chain can be fused with a seqence encoding the heavy chain to form a single-chain antibody.

For example, the DNA molecule of the present invention may contain VH encoding nucleotide sequence as set forth in SEQ ID NO: 3 or SEQ ID NO: 7; and VL encoding nucleotide sequence as set forth in SEQ ID NO: 5 or SEQ ID NO: 9. For example, the encoding nucleotide sequence of c23C3 VH is shown in SEQ ID NO: 3, and the encoding nucleotide sequence of c23C3 VL is shown in SEQ ID NO: 5; the encoding nucleotide sequence of h23C3 VH is shown in SEQ ID NO: 7, and the encoding nucleotide sequence of h23C3 VL is shown in SEQ ID NO: 9.

Once the relevant sequence is obtained, we can use recombination methods to get a large amount of relevant sequences. Usually, the obtained relevant sequence is cloned into vectors to be transferred to host cells, and then isolated from the proliferated host cell by conventional method.

In addition, the relevant sequence can be synthesized by artificial synthesis methods, especially for short ones. Typically, multiple short fragments are first synthesized, which are then connected into a long sequence.

At present, the DNA sequence encoding the anti-hOPN monoclonal antibody (or the fragments or derivatives thereof) of the present invention can be obtained completely through chemical synthesis. Then the DNA sequence can be introduced into a variety of DNA molecules (or vectors) and cells known in the art. In addition, mutations can be introduced into the sequence of proteins according to the present invention by chemical synthesis.

The present invention also involves vectors containing the above DNA sequence and a suitable promoter or other regulatory sequence(s). These vectors can be used to transform suitable host cells to express the protein. The host cell can be prokaryotic cells, such as bacterial cell; or lower eukaryotic cells, such as yeast cells; or higher eukaryotic cells, such as mammalian cells.

The invention also provides a hybridoma cell line that can produce the anti-hOPN monoclonal antibodies of the present invention. Preferably, the present invention provides a hybridoma cell line that can produce the anti-hOPN monoclonal antibodies having high efficiency.

Upon obtaining the hybridoma which can produce the anti-hOPN monoclonal antibody of the present invention, the skilled in the art can easily determine the structure of the antibody of the invention (such as the VH and the VL of the antibody), and then prepare the monoclonal antibody of the invention (for example) in the ways as described below.

An expression vector which contains the nucleotide sequence of the monoclonal antibody of the invention and expression-regulatory sequences operably linked to said sequence is first constructed.

As used herein, the term "expression-regulatory sequence" usually refers to a sequence contributing to the regulation of the expression of nucleotide sequences. The expression-regulatory sequences include promoters and termination signals operably linked to target nucleotide sequences. Usually, expression-regulatory sequences also include the sequences which are need for the proper translation of the nucleotide sequences. The term "Operably linked" refers to certain parts of a linear DNA sequence which can affect the activity of other parts of the same linear DNA sequence. For example, if a promoter or enhancer increases the transcription of an encoding sequence, then it is operably linked to the encoding sequence.

The DNA sequences encoding the present monoclonal antibody can be produced by well-known techniques in the art. For example, the VH and VL encoding nucleotide sequences of the monoclonal antibody can be artificially synthesized or amplified by PCR based on the herein disclosed sequences. Then, various methods known in the art can be used to introduce these nucleotide sequences into appropriate expression vectors by selecting appropriate enzyme cleavage sites so as to make them separately located ahead of the CH encoding sequence and CL encoding sequence carried by the expression vector, and in the same reading frame of the CH and CL encoding sequences. The expression vectors used in the present invention are well-known and commercially available, such as pPICZα and pPIC9K. The expression vectors containing the above nucleotide sequences of the present invention can be, for example, pcDNA3.1/ZEO (+) and pcDNA3.1 (+).

The above obtained expression vector is used to transform suitable host cells. The term "host cell" generally includes prokaryotic cells and eukaryotic cells. Examples of commonly used prokaryotic host cells include *E. coli, Bacillus subtilis* and so on. Commonly used eukaryotic host cells include yeast cells, insect cells, and mammalian cells. In the invention, mammalian cell is preferred. Mammalian cell line is usually used as host cell to express eukaryotic cell-derived peptides. The proliferation of mammalian cells in culture is in well known in the art. See "Tissue Culture", Academic Press, Kruse and Patterson Ed. (1973), the disclosures of which are hereby incorporated by reference. Preferred mammalian cells are commercially available immortalized cell lines. These immortalized cell lines include but not limited to the Chinese hamster ovary (CHO) cells, the Vero cell, the Hela cells, the baby hamster kidney (BHK) cells, the monkey kidney cells (COS), the human hepatocellular carcinoma cells (such as Hep G2) and many other cell lines. They provide post-translation modification to the proteins, including correct folding, correct formation of disulfide bond and correct glycosylation sites.

There are many methods for transforming host cell with expression vector, and the transformation procedure used depends on the host to be transformed. The methods for introducing heterologous polynucleotide into mammalian cells are well known in the art, which includes dextran-mediated transfection, calcium phosphate precipitation, Polybrene (1,5-dimethyl-1,5-diazaundecamethylene polymethobromide)-mediated transfection, protoplast fusion, electroporation, and liposome-mediated transfection, as well as direct DNA microinjection into the nucleus. In the present invention, the preferred method is electroporation, or liposome-mediated transfection. For example, the Invitrogen liposome transformation kit can be used to transfect host cells such as CHO cells and so on. In the present invention, the preferred host cell is COS or CHO cell.

The transformed host cell is then cultured under conditions suitable for the expression of the monoclonal antibody of the present invention. Then the humanized anti-hOPN monoclonal antibody of the invention can be obtained using conventional immunoglobulin purification steps, such as protein A-Sepharose, hydroxyapatite chromatography, gel electrophoresis, dialysis, ion-exchange chromatography, hydrophobic chromatography, molecular sieve chromatography, or affinity chromatography and other well known conventional separation and purification methods.

### Identification, expression and purification of monoclonal antibody

The resultant monoclonal antibody can be identified by conventional procedures. For example, the binding specificity of the monoclonal antibody can be determined by immunoprecipitation *or in vitro* binding assay (such as radioimmunoassay (RIA) or enzyme-linked immunosorbent assay (ELISA)). The binding affinity of the monoclonal antibody can be determined by, for example, the Scatchard analysis of Munson, et al., Anal. Biochem., 107:220 (1980).

The anti-hOPN monoclonal antibody of the present invention can be expressed in the cell, or expressed on the surface of the cell, or can be secreted to the outside. If necessary, the recombinant proteins can be separated and purified using a variety of separation methods based on their physical, chemical, and other features. These methods are well known in the art. Examples of these methods include, but not limited to, conventional renaturation treatment, treatment with the protein precipitating agent (salting-out method), centrifugation, osmosis, ultrasound treatment, ultracentrifugation, molecular sieve chromatography (gel filtration), adsorption chromatography, ion-exchange chromatography, high performance liquid chromatography (HPLC) and various other liquid chromatography techniques and their combinations.

### Pharmaceutical composition

The present invention also provides a pharmaceutical composition for the treatment of autoimmune disease, which comprises pharmaceutically effective amount of the monoclonal antibody of the invention or immunoconjugate thereof, as well as pharmaceutically acceptable carriers.

As used herein, the term "pharmaceutically acceptable" refers to the property of a molecular or combination comprising the same that when administering to animals or human, the molecular itself and combinations thereof will not cause disadvantaged, allergic or other adverse reactions. As used herein, the term "pharmaceutically acceptable carrier" should be compatible with the active components of the present invention, that is, can be combined with the active components and usually will not significantly reduce the effect of the pharmaceutical composition. These carriers are well known in the art. A thorough discussion of the pharmaceutically acceptable carriers can be found in Remington's Pharmaceutical Sciences (Mack Pub. Co., NJ 1991).

Such carriers include, but not limited to: saline, buffers, glucose, water, glycerin, ethanol, adjuvants, and combinations thereof. In addition, these carriers may also comprise auxiliary substances, such as wetting agents or emulsifying agent, pH buffering agents and so on.

The composition of the invention can be administered via conventional manners such as via oral, intravenous injection, intramuscular injection or subcutaneous injection, preferably via oral or intravenous administration is preferred.

Typically, the pharmaceutical composition of the present invention comprises 0.00001 - 99.9 wt%; preferably 0.0001 - 90wt%, more preferably 0.001 - 75wt%, even more preferably 0.01 - 50wt% of the monoclonal antibody of the present invention or immunoconjugate thereof, based on the total weight of the composition. The rest of the composition is consisted of pharmaceutically acceptable carrier(s) and other additive(s).

The pharmaceutical compositions of the invention can be formulated into a variety of dosage forms according to particular needs, and can be administered in an effective amount by a physician according to the factors, such as the type, age, body weight and general conditions of the patient, and routes of administration. The route of administration can be, for example, infusion and other therapeutical ways.

When applying the pharmaceutical composition, a safe and effective amount of anti-hOPN monoclonal antibody or immunoconjugate is administered to mammals, wherein the safe and effective amount is usually about 0.1 µg-5 mg/kg body weight, and in most cases, not more than about 5 mg/kg body weight, preferably in the range of about 1-10 µg/kg body weight to about 1 mg/kg body weight. Of course, when determining the particular dosage, the factors such as administration routes and the health status of the patient should also be taken into consideration, which are within the skills of the practiced physicians.

The monoclonal antibody or immunoconjugate of the invention can be used for the treatment of autoimmune diseases, in which osteopontin acts as an important pathogenic factor, by targeting osteopontin. Such autoimmune diseases including, but not limited to: rheumatoid arthritis, multiple sclerosis, autoimmune hepatitis, ankylosing spondylitis, Crohn's disease, rheumatoid spondylitis, osteoarthritis, gouty arthritis, autoimmune diabetes, autoimmune uveitis, nephrotic syndrome, ulcerative colitis, psoriatic arthritis, undifferentiated spondyloarthropathy, chronic liver failure, and other conditions relating to rheumatoid arthritis including weight gain, joint distortion, joint swelling, joint deformity, joint bending stiffness, severe movement disorders, and combinations thereof.

The pharmaceutical composition of the invention can also comprise the substances having therapeutic or ameliorative activity on autoimmune disease, or can be used in combination with other active agents, to obtain improved therapeutic effects. The other substances having therapeutic or ameliorative activity on autoimmune disease include, but not limited to: anti-inflammatory cytokines including IL-4, IL-6, IL-10 and/or IL-4 antagonist, ENBREL®, phosphodiesterase IV inhibitors, anti-inflammatory inhibitors and cytokine inhibitors such as leflunomide, non-steroidal inflammatory drugs such as naproxen, cortisol, or cyclosporine.

When two or more drugs are administrated in combination, the effect thereof is usually better than the effect produced by separately administrating the individual drugs. Preferably, the drugs or other agents administrated in combination with the monoclonal antibody of the present invention do not interfere with the therapeutic activity of said monoclonal antibody.

### Kit for the Detection of hOPN

The present invention also provides a kit for the detection of hOPN, which contains anti-hOPN monoclonal antibody as well as the active fragment or immunoconjugate thereof. The kit of the present invention can be used to detect the presence or content of osteopontin in the biological sample. The method for detection comprises the following steps: (i) contacting the biological sample with the anti-osteopontin monoclonal antibody or immunoconjugate of the invention; (ii) detecting the formation of antigen - antibody complex, wherein the formation of antigen - antibody complex suggests the presence of osteopontin in the sample, or quantitatively determining the content of the formed antigen - antibody complex so as to reflect the content of osteopontin in the sample. The sample can be pre-treated or not, for example by extraction, purification, or enrichment.

The kit comprises container and the monoclonal antibody of the present invention contained in the container, or plate(s) used for detection containing said monoclonal antibody, and instruction. The kit can also comprise other reagent(s) needed for detection, such as buffer, indicators and so on. The skilled in the art can adjust the contents in the kit according to particular need.

### Examples

The invention will be further described in the following examples and experiments, which do not limit the scope of the invention. Examples do not include a detailed description of conventional methods, such as those used to construct vectors and plasmids, to insert protein encoding genes into such vectors and plasmids, to introduce plasmids into host cells, or the classic cell fusion method and monoclonal antibody screening and purification methods. Such methods are well known in the art and have been described in many publications, including Sambrook, J., Fritsch, E. F. and Maniais, T. (1989) "Molecular Cloning: A Laboratory Manual", Cold spring Harbor Laboratory Press. All percentages and parts are based on weight, unless otherwise indicated. All the materials and adjuvants in the present examples or experiments are commercially available, unless specially indicated.

### Example The preparation of mouse anti-human OPN monoclonal antibody

### Example 1. Human OPN cDNA fragment cloning

The following primers were synthesized based on human OPN information and sequence provided in GENEBANK: OPN sense primer (primer 1): GGG AAGCTT ACCATGAGAATTGCAGTGATTTG (Hind III) (SEQ ID NO: 16); and OPN antisense primer (primer 2): GCC GGTACC ATTGACCTCAGAAGATGCAC (Kpn I) (SEQ ID NO: 17). Human liver cancer cell line LM3 (purchased from Shanghai Zhongshan Hospital) was cultured and proliferated before extracting RNA by RT-PCR (PROMEGA) using TRISOL kit (INVITROGEN). The PCR condition was: 94°C for 5 mins; 30 cycles of 94°C for 45 sec, 58°C for 30 sec, and 72°C for 45 sec; and 72°C for 10 mins. The resultant DNA fragment having 963bp was recovered by gel extraction kit (Shanghai Sangon) and cleavaged with restriction enzymes Hind III and Kpn I. The cleavaged fragment was recovered by gel electrophoresis and was linked to the Hind III and Kpn I double-digested plasmid vector. The vector was then transformed into *E. coli* DH10B, and the positive clones with inserts were screened. The DNA sequence was confirmed by sequencing. The nucleotide sequence of OPN gene was as set forth in SEQ ID NO: 1.

### Example 2. Mouse OPN cDNA fragment cloning

The following primers were synthesized based on mouse OPN information and sequence provided in GENEBANK: mouse OPN sense primer (primer 3): ATAAGCTTGGATGACGACGACAAGATGAGAATTGCAGTGATT (Hind III) (SEQ ID NO: 18) and mouse OPN antisense primer (primer 4): ATCTCGAGTTAATTGACCTCAGAAGA (Kpn I) (SEQ ID NO: 19). The mouse spleen T lymphocyte was separated and activated with ConA for 30 hours. Then, RNA was extracted by RT-PCR (PROMEGA) using TRISOL kit (INVITROGEN). The PCR condition was: 94°C for 5 mins; 30 cycles of 94°C for 45 sec, 55°C for 30 sec, 72°C for 45 sec; and 72°C for 10 mins. The target DNA fragment was obtained, revocered by gel extraction kit (Shanghai Sangon), and then cleavaged with restriction enzymes Hind III and Kpn I. The cleavaged fragment was recovered by gel electrophoresis and was linked to the Hind III and Kpn I double-digested plasmid vector. The vector was transformed into *E. coli* DH10B, and the positive clones with inserts were screened. The DNA sequence was confirmed by sequencing. The nucleotide sequence of mouse OPN gene was as set forth in SEQ ID NO: 2.

### Example 3. Eukaryotic cell expression and purification of human and mouse OPN

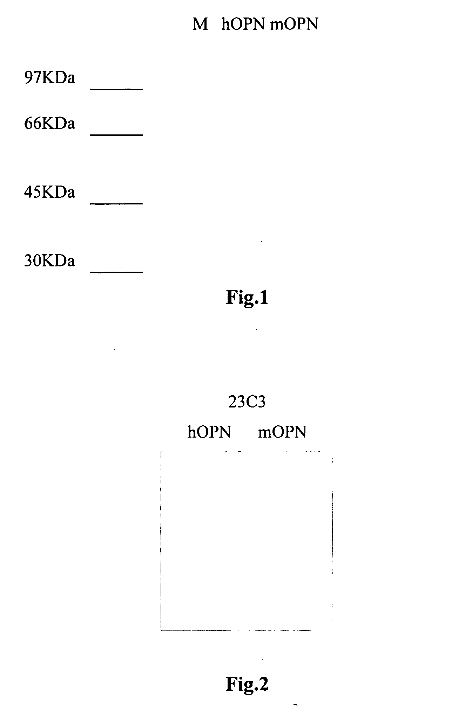

The correct human and mouse OPN gene fragments obtained in example 1 and example 2 were recovered after corresponding restriction enzyme digestion, and introduced into pPICZα plasmid. *Saccharomyces Pichia* cell was transfected with the plasmid, and individual clone was picked out and induced to express human and mouse OPN proteins. The expression supernatant of yeast cell was collected and purified with anion exchange and molecular sieve. The result of SDS-PAGE proved the obtained proteins to be pure human and mouse OPN proteins. Then the purified proteins were subjected to SDS-PAGE electrophoresis, and the result was shown in Figure 1.

### Example 4. Screen and preparation of mouse anti-human OPN monoclonal antibody

One hundred µg of human OPN and an equal volume of Freund's adjuvant were emulsified, and the resultant was administrated to BALB/C mouse (i.p.). The mice were boosted every two weeks with the same dose. After three immunizations, the mice with relatively high serum anti-OPN antibody titer were selected. The spleen lymphocytes of the selected mouse were separated and fused with NS-1 cells using classic PEG method. 96-well plate coated with 10µg/ml OPN was used for repeated screening by ELISA method to obtain hybridoma cell line 23C3 which was capable of stably expressing anti-human OPN antibody. The monoclonal cell line 23C3 was amplified, and administrated to BALB/C mice in 5 × 10⁶ cells/mouse (i.p.). Mouse ascites were collected after 10 days, and anti-human OPN monoclonal antibody was purified by affinity chromatography using protein A column. The result of western blot showed that mouse anti-human OPN monoclonal antibody 23C3 not only specifically bound with human OPN protein, but also cross acted with mouse OPN protein. The result was shown in Figure 2. The anti-OPN antibody was commercially available (R & D company)

### Example 5. Cloning and sequencing of 23C3 variable region gene

23C3 (5 × 10⁶ - 1 × 10⁷) hybridoma cell were collected, and total RNA were extracted using TRIzol (Invitrogen, Catalog No. 15596-026). The following primers were designed according to mouse antibody constant region sequences:
HGSP1: 5'-GATACTGTGATCTGTTTG-3'(SEQ ID NO: 20)
HGSP2: 5'-TCGCAGATGAGTCTGGAC-3 '(SEQ ID NO: 21)
HGSP3: 5'-ATGAACACACTCACATTG-3 '(SEQ ID NO: 22)
LGSP1: 5'-GAGGTTATGACTTTCATAGTCAGC-3 '(SEQ ID NO: 23)
LGSP2: 5'-AACACTGTCCAGGACACCATCTCG-3 '(SEQ ID NO: 24)
LGSP3: 5'-TCTGGGATAGAAGTTGTTCATGAG-3 '(SEQ ID NO: 25)

By using Invitrogen 5'RACE kit (Catalog No. 18374-058), the first-strand cDNA was synthesized using the primers HGSP1 and LGSP1 respectively; Poly C was added to 3' terminal of the first-strand cDNA using TdT and dCTP; the PCR products of the VH and VL were obtained using HGSP2, HGSP3, LGSP2 and LGSP3 as 5' primer in nested-PCR; The PCR products were introduced into pGEM-T vector. Clones were picked out to extract plasmid, and the positive clones were identificated by using restriction enzymies and then subject to sequencing. The nucleotide and amino acid sequences of the VH in 23C3 were as set forth in SEQ ID NO: 3 and SEQ ID NO: 4 respectively, and the nucleotide and amino acid sequences of the VL in 23C3 were as set forth in SEQ ID NO: 5 and SEQ ID NO: 6 respectively.

### Example 6. Cloning of the light chain and heavy chain constant region genes of human antibody

Healthy human lymphocytes were isolated using lymphocyte separation medium (DingGuo Biotechnology Development Co.). Total RNA was then extracted using Trizol reagent (Invitrogen). Primers were designed according to the sequences reported in Cell, 1980, 22: 197-207 and Nucleic Acids Research, 1982, 10: 4071-4079, and antibody heavy chain and light chain constant region genes were amplified by RT-PCR. PCR products were purified and collected by agarose gel electrophoresis and cloned into pGEM-T vector, verified by sequencing to obtain correct clone. The nucleotide sequence and amino acid sequence of the heavy chain constant region (CH) were as set forth in SEQ ID NO: 12 and SEQ ID NO: 13, respectively. The nucleotide sequence and amino acid sequence of the light chain constant region (CL) were as set forth in SEQ ID NO: 14 and SEQ ID NO: 15. The correct clones of this example were known as pGEM-T/CH and pGEM-T/CL.

### Example 7. Construction of anti-OPN chimeric antibody c23C3

The chimeric antibody heavy chain gene was synthesized by overlap PCR using the VH genes 23C3VH of 23C3 and pGEM-T/CH vector as template. The reaction condition was: 95°C for 15 mins; 30 cycles of 94°C for 50 sec, 58°C for 50 sec, and 72°C for 50 sec; 72°C for 10 mins. The chimeric heavy chain gene had a Hind III restriction enzyme site and a signal peptide gene sequence at 5' end, and a translation stop codon TAA and an EcoRI restriction enzyme site at 3' end. The sequence of the signal peptide gene was ATGGATTTTCAGGTGCAGATTTTCAGCTTCCTGCTAATCAGTGCCTCAGTCAT AATATCCAGAGGA (SEQ ID NO: 52). PCR amplified product was separated by agarose gel electrophoresis. The target band was recovered and cloned into pGEMT vector. Positive clone was screened and sequenced. The correct clones identified by sequencing were digested with Hind III and EcoR I. Chimeric antibody heavy chain fragment c23C3VHCH was purified and recovered via agarose gel electrophoresis, linked to Hind III and EcoR I double-digested plasmid pcDNA3.1 (+) (Invitrogen, USA), and constructed into chimeric heavy chain eukaryotic expression vector pcDNA3.1 (+) (c23C3VHCH).

The light chain gene of the chimeric antibody was synthesized by overlap PCR using the VL gene 23C3VL of 23C3 and pGEM-T/CL vector as template. The reaction condition was: 95°C for 15 mins; 30 cycles of 94°C for 50 sec, 58°C for 50 sec, and 72°C for 50 sec; 72°C for 10 mins,. The obtained PCR product had a Hind III restriction enzyme site and a signal peptide gene sequence at 5' end, and a translation stop codon TAA and an EcoR I restriction enzyme site at 3' end. The signal peptide gene sequence was ATGGATTTTCAGGTGCAGATTTTCAGCTTCCTGCTAATCAGTGCCTCAGTCAT AATATCCAGAGGA (SEQ ID NO: 52). The PCT products were recovered by agarose gel electrophoresis and cloned into pGEM-T vector (Promega). Positive clones were screened and sequenced. The clones with correct sequence were picked out and digested with Hind III and EcoR I. The light chain fragments c23C3VLCL of the chimeric antibody were purified and recovered by agarose gel electrophoresis, linked to Hind III and EcoR I double-digested plasmid pcDNA3.1/ZEO(+) (Invitrogen, USA), and constructed into chimeric light chain eukaryotic expression vector pcDNA3.1/ZEO(+) (c23C3VLCL).

The successfully constructed chimeric heavy chain and light chain expression vectors were used to co-transfect COS-1 cell (ATCC CRL 1650) by liposomal transfection method. The supernatant was collected for analysis after 72 hours. The content of chimeric antibody c23C3 in supernatant was determined by ELISA. ELISA plate was coated with goat anti-human IgG (Fc), and blocked with 2% BSA-PBS at 37°C for 2 hours. The supernatant to be tested and the standard (Human myeloma IgG1, k) were added into the plate and incubated at 37°C for 2 hours. HRP-goat anti-human κ was then added to perform binding reaction at 37°C by incubated for 1 hour. TMB was added and incubated at 37°C for 5 minutes before adding H₂SO₄ to terminate the reaction. OD450 was measured.

### Example 8. Homology modeling of the three-dimensional structure for murine 23C3 monoclonal antibody variable regions (Fv)

Insight II package (Accelrys) was used to simulate the three-dimensional structure for 23C3 murine monoclonal antibody variable region. First, the template proteins for the HL and VL proteins of 23C3 were searched in Protein Data Bank (PDB) using BLAST program. Antibody 1cbv and 1F6L with highest homology were selected as the modeling templates for 23C3, and the three-dimensional structure of 23C3 was constructed using Insight II program (Figure 28).

### Example 9. Design and construction of humanized anti-OPN antibody

BLAST program was used to search the most similar human origin templates for the VL and the VH of 23C3 respectively in Genebank database. The human origin antibody which has the highest homologous to the VH of 23C3 was human antibody AAT51715.1 (gb | AAT51715.1 |), with a similarity of 72%, and the human origin antibody which has the highest homology to the VL of 23C3 was BAC01726.1 (dbj | BAC01726.1 |), with a similarity of 72%. Therefore, AAT51715.1 and BAC01726.1 were used as the templates for humanizing 23C3 heavy chain and light chain.

The CDRs in the heavy chain and light chain of 23C3 were transplanted directly into human origin template AAT51715.1 and BAC01726.1 respectively to construct a CDR graft antibody, which had heavy chain h23C3Ha and light chain h23C3La. The sequences of h23C3Ha and variable region sequences were shown in Figure 29. The HV and VL genes (h23C3VHa and h23C3VLa) of humanized antibody were whole gene synthesized, and then the heavy chain gene of the humanized antibody was synthesized via overlap PCR by using h23C3VHa gene and pGEM-T/CH vector as templates. The reaction condition was: 95°C for 15 mins; 30 cycles of 94°C for 50 sec, 58°C for 50 sec, and 72°C for 50 sec; 72°C for 10 mins. The humanized antibody heavy chain gene was made to have Hind III restriction enzyme site and signal peptide gene sequence at 5' end and have translation stop codon TAA and EcoRI restriction enzyme site at 3' end. The sequence of signal peptide gene was ATGGATTTTCAGGTGCAGATTTTCAGCTTCCTGCTAATCAGTGCCTCAGTCAT AATATCCAGAGGA (SEQ ID NO: 52). The products amplified by PCR were separated by agarose gel electrophoresis, and the target band was recovered and cloned into pGEMT vector. Positive clones were screened and sequenced. The clones having correct sequence were selected and digested with Hind III and EcoR I. The heavy chain fragment of the humanized antibody was purified and recovered by agarose gel electrophoresis, linked to Hind III and EcoR I double-digested plasmid pcDNA3.1 (+) (Invitrogen, USA) by T4 DNA ligase (Invitrogen) to construct a humanized heavy chain eukaryotic expression vector.

Humanized antibody light chain gene was synthesized by overlap PCR by using h23C3VLa gene and pGEM-T/CL vector as templates. The reaction condition was: 95°C for 15 mins; 30 cycles of 94°C for 50 sec, 58°C for 50 sec, 72°C for 50 sec; 72°C for 10 mins. The obtained PCR product, h23C3VlaCL, had a Hind III restriction enzyme site and a signal peptide gene sequence at 5' end, and a translation stop codon TAA and an EcoRI restriction enzyme site at 3' end. The sequence of signal peptide gene was ATGGATTTTCAGGTGCAGATTTTCAGCTTCCTGCTAATCAGTGCCTCAGTCAT AATATCCAGAGGA (SEQ ID NO: 52). The clones with correct sequence were picked out and digested with Hind III and EcoR I. The light chain fragment the humanized antibody agarose gel electrophoresis, and linked to Hind III and EcoR I double-digested plasmid pcDNA3.1ZEO(+) (Invitrogen, USA) to construct a humanized light chain eukaryotic expression vector pcDNA3.1/ZEO (+) (h23C3VLaCL).

The successfully constructed heavy chain and light chain expression vectors were used to co-transfect COS-1 cell (ATCC CRL 1650) by liposome transfection method. The supernatant was collected for analysis after 72 hours. The content of humanized antibody (H23C3Ha/H23C3La) in the supernatant was determined by ELISA. ELISA plate were coated with goat anti-human IgG (Fc), and blocked with 2% BSA-PBS at 37°C for 2 hours. The supernatant to be tested and the standard (Human myeloma IgG1, κ) were added into the wells, and incubated at 37°C for 2 hours. HRP-goat anti-human κ was then added for the binding reaction (7°C incubated for 1 hour). TMB was then added and incubated at 37°C for 5 minutes before adding H₂SO₄ to terminate the reaction. OD450 was measured.

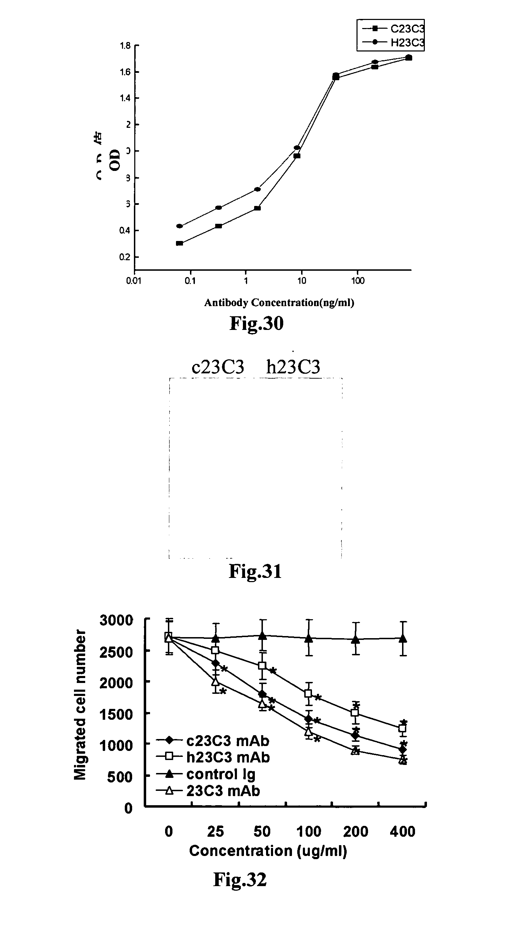

ELISA plate was coated with 2 ug/ml OPN. The binding activity of transfected humanized antibody to OPN was detected by ELISA. The result showed that the activity of humanized antibody composed of H23C3Ha and H23C3La (H23C3a / H23C3La) was almost lost when comparing with C23C3 chimeric antibody. Therefore, in order to obtain humanized antibody with high-affinity, murine FR residues which might affect h23C3 antibody binding activity should be subject to analysis and reverse mutation. By analyzing the three-dimensional structure of modeled 23C3 monoclonal antibody variable region (Figure 28), we found that within 5Å spatial area around CDR, there were seven residues in the FR region which may affect the conformation of original antibody CDR and had different positions to the corresponding residues in human source template. The residues were L1Tyr, L45Gln, L48Val, L70Gln, H30Asn, H49Ala and H93Val. Retaining those murine amino acid residues in the constructed CDR graft antibody could produce a humanized antibody (h23C3Hb/h23C3Lb). The amino acid sequences of h23C3Hb and h23C3Lb variable region were shown in Figure 29. SEQ ID NO: 7 and SEQ ID NO: 8 showed the nucleotide sequence and amino acid sequence of h23C3Hb variable region, respectively. SEQ ID NO: 9 and SEQ ID NO: 10 showed the nucleotide sequence and amino acid sequence of h23C3Lb variable region, respectively. The whole genes of the VH and the VL in the humanized antibody (h23C3VHb/h23C3VLb) were synthesized. The light chain expression vector pcDNA3.1/ZEO (+) (VH23C3VLbCL) and heavy chain expression vector pcDNA3.1 (+) (VH23C3VHbCH) were constructed using the same method as that used for constructing the humanized antibody (h23C3Ha/h23C3La). Then the light and heavy chain expression vectors were used to cotransfect COS-1 cell. The antibody-antigen binding activity was determined by ELISA method. ELISA plate was coated with OPN protein (2ug/ml) at 4°C overnight, and blocked with 2% BSA-PBS at 37°C for 2h, The supernatant of H23C3 culture to be tested was added into the plate, and the plate was incubated at 37°C for 2h. Then HRP-rabbit anti-human IgG was added into the plate for binding reaction (incubated at 37°C for 1h). TMB was then added and reacted at 37°C for 5min before adding H₂SO₄ to terminate the reaction. A450 and A630 were measured. The result of H23C3 specific binding to OPN was shown in Fig. 30.

The binding activity of H23C3 to OPN was similar to that of c23C3 chimeric antibody, and the humanized antibody (h23C3Hb/h23C3Lb) was named as h23C3.

### Example 10. The stable expression and purification of anti-OPN humanized antibody h23C3

The successfully constructed c23C3 or h23C3 heavy chain and light chain plasmids were used to co-transfect CHO-K1 cell (ATCC CRL-9618) by liposome transfection method. After 24 hours, the media was changed to a selective media comprising 600µg/ml G418 and 250µg/ml Zeocin for the selection of resistant clones. The supernatant of the cell culture was collected and was screened for high expression clones by ELISA assay. The selected high expression clones were expanded in serum-free culture medium. Chimeric antibody c23C3 or humanized antibody h23C3 were isolated and purified by protein A affinity column (GE), and were finally quantified by UV absorption.

### Example 11. Determination of the binding ability of chimeric antibody c23C3 and humanized antibody h23C3 to OPN

SDS-PAGE (10%) electrophoresis was carried out (5ug hOPN/lane). hOPN was transferred to nitrocellulose membrane by 360 mA constant current for 1 hour. The membrane was blocked with 10% skimmed milk at 4°C overnight or incubated at room temperature for two hours; washed with 1 × TBST (Tween-20, 0.1%) for 3 times (10 minutes per time); reacted with 10ug/ml c23C3 and h23C3 respectively at room temperature for 1 hour, washed with 1 × TBST (Tween-20, 0.1%) for five times, 10 minutes per time; and then reacted with 1:1000 diluted HRP labeled mouse anti-human IgG (Beijing Zhongshan Company) at room temperature for 1 hour, treated with ECL kit (Tiangen Company) for 1-2 minutes, pressed and exposed with medical blue-sensitive X-ray film. The result was shown in Figure 31. The results suggest that c23C3 and h23C3 are both capable of specifically binding with hOPN.

### Experiment I. Effect of 23C3 monoclonal antibody on CIA arthritis model mice

### Experiment I-1. Effect of 23C3 mAb treatment on clinical scores for arthritis, onset time and incidence rate in CIA arthritis model mice

One hundred µg of chicken type II collagen (Condrex) and equal volume of Freund's complete adjuvant were emulsified, injected subcutaneously into DBA/1J mice (32 mice, male, 8-10 weeks old) at the tail for primary immunization. The mice were boosted after 21 days, i.e. 100 µg of chicken type II collagen and equal volume of Freund's complete adjuvant were emulsified and injected subcutaneously at the tail for boosting. The mice which had been immunized for two times, were divided into four groups and administrated with control antibody (mouse IgG, 200 µg/mouse, Sigma), and 23C3 monoclonal antibody (200 µg/mouse, 100 µg/mouse, and 50 µg/mouse) respectively. All the treatment groups were administered by intraperitoneal injection, and administered every other day for six times from the day of the second immunization. The clinical scores for arthritis, onset time and incidence rate were observed from the day of the second immunization and for 30 days. The arthritis severity in mice was scored with reference to the literature ("A Role for the Lymphotoxin/LIGHT Axis in the Pathogenesis of Murine Collagen-Induced Arthritis". Roy A. Fava, Evangelia Notidis, Jane Hunt, Veronika Szanya, Nora Ratcliffe, Apinya Ngam-ek, Antonin R. de Fougerolles, Andrew Sprague, and Jeffrey L. Browning. In particular, each leg was scored as 0-4 according to the severity of inflammation, wherein 0 - no swelling; 1 - swelling in one finger or toe, or slight edema; 2 - swelling in more than one fingers or toes, or moderate edema; 3 - swelling in most of the fingers or toes, or severe edema; 4 - swelling in all of the fingers or toes, or deformity. The clinical scores for arthritis in mice was the sum of the inflammation scores of fore limbs and hind limbs.

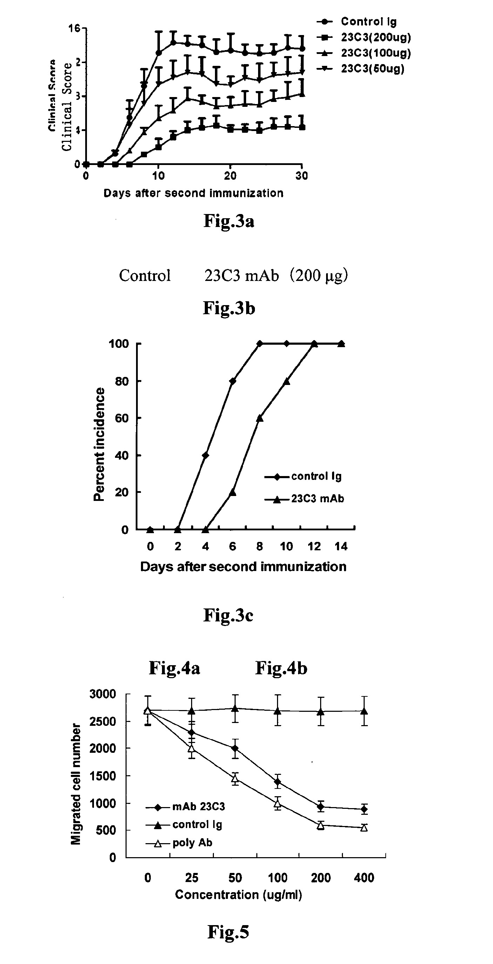

The experimental results were shown in Figure 3a, 3b, and 3c. 23C3 monoclonal antibody could significantly reduce the clinical scores for arthritis, delay the onset time, but had no significant effect on incidence rate when comparing to control group.

### Experiment I-2. The protective effect of 23C3 mAb treatment on joint bone resorption in CIA mouse

The CIA mice in the group receiving 200 µg 23C3 monoclonal antibody treatment and the control group (as indicated in Experiment I-1) were subject to X-ray detection at the 30^{th} day after the second immunization to observe the bone destruction of the sick joints. The result was shown in Figure 4, as compared to the treatment group, the joint bone resorption in the phalangeal joints of the mice of 23C3 monoclonal antibody treatment group was not obvious. The results suggest that 23C3 monoclonal antibody has a good protective effect on bone resorption.

### Experiment I-3. The in vitro inhibitory effect of 23C3 mAb on lymphocyte migration

The spleens of the CIA mice (experiment I-1) were aseptically separated, passed 100 mesh steel screen, washed with PBS for two times, and treated with lymphocyte separating medium to separate spleen lymphocytes, and the resultant was centrifuged at 2000 rpm for 20 minutes. The lymphocyte layer was pipetted and washed with PBS for 2 times. The lymphocytes were counted, and adjusted to a concentration of 5 × 10⁶ cells/ml. 24-well tissue culture plate having a 5 µm filter membrane at the upper layer was pre-coated with 10ug OPN at the lower layer. 5 × 10⁵ lymphocytes as well as the following antibodies were added into the upper layer of each well: 23C3 (anti-OPN monoclonal antibody), Her-2 antibody (irrelevant antibody control), and anti-OPN polyclonal antibody (CHEMICON). The plate was incubated at 37°C, 5% CO₂ for 2 hours, and then washed with PBS twice. The number of cells in the lower layer was calculated by taking the average of the counted numbers in five high power fields and then multiplied by 50.

The experimental result was shown in Figure 5. 23C3 mAb could effectively block the migration of lymphocytes in a dose dependent manner.

### Experiment I-4. OPN level changes in the plasma and joint tissue of CIA mice in different treatment groups

The DBA/1J mice plasma of different time points (0, 7, 14 or 28 days) in different treatment groups (experiment I-1) were collected, and detected for the OPN protein concentration using mouse OPN ELISA kit (R & D Company). Specifically, venous blood from mice outer canthus was collected, and centrifuged immediately at 4000 rpm for 10 minutes to separate the plasma. The 1:200 dilution was added into the 96 well plate of ELISA detection kit. The plate was incubated at room temperature for 1 hour, and washed with PBST for five times. Biotin-labeled anti-OPN primary antibody was then added into the plate before incubating the plate at room temperature for 1 hour. After the plate was washed for five times, avidin labeled secondary antibody was added into the plate. The plate was incubated for 1 hour at room temperature, washed for six times, and developed with TMB substrate. OD450 absorbance was detected. The plasma OPN concentration was calculated according to the standard curve.

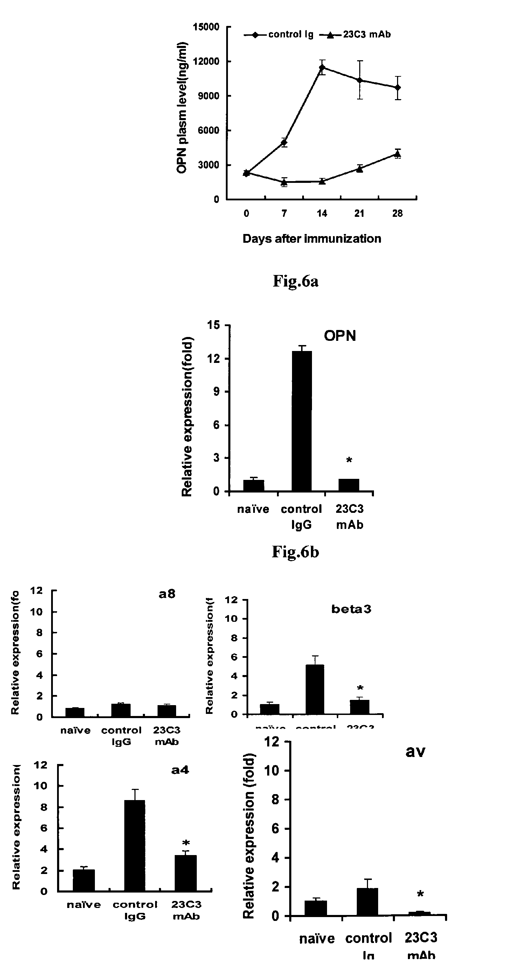

The result was shown in Figure 6a. OPN level in control group was much higher than that in 23C3 treatment group, and reached the highest level at day 14, which was 10 times that of 23C3 treatment group. The result suggests that OPN protein level may be related to the occurrence and severity of the disease, and the reduction of OPN level can reduce arthritis severity.

At day 14, the joint tissues of CIA mice in different treatment groups of experiment I-1 were collected. The skin and muscle tissues were removed. Total RNA was extracted with RNeasy Mini Kit (QIAGEN) and subjected to reverse transcription with Sensiscript RT Kit (QIAGEN) for cDNA. OPN primers were designed according to the requirements of real-time PCR (as shown in Table 1). OPN mRNA level was detected by real-time PCR using SYBR Green Master Mix (Applied Biosystems). The PCR procedure was: 50°C for 2 mins; 95°C for 10 mins; 40 s of 95°C for 15 sec and 60°C for 60 sec. The internal standard was GAPDH gene. The experimental data was analyzed using ABI PRISM 7900 sequencing system (Applied Biosystems).

The experimental results were shown in Figure 6b. The OPN level in the joint tissue of 23C3 monoclonal antibody treatment group was much lower than that of the control group, and even lower in 23C3 treatment group. The results suggest that 23C3 can regulate OPN mRNA level by neutralizing tissue OPN protein.

### Experiment I-5. The mRNA levels of OPN receptors in the joint tissue of CIA mice in different treatment groups

At day 14, the joint tissues of CIA mice in different treatment groups of experiment I-1 were collected. The skin and muscle tissues were removed. Total RNA was extracted with RNeasy Mini Kit (QIAGEN) and subjected to reverse transcription with Sensiscript RT Kit (QIAGEN) for cDNA. Primers for OPN receptors were designed according to the requirements of real-time PCR (as shown in Table 1). OPN receptor mRNA level was detected by real-time PCR using SYBR Green Master Mix (Applied Biosystems). The PCR procedure was the same as that used in experiment 4. The experimental data was analyzed using ABI PRISM 7900 sequencing system (Applied Biosystems).

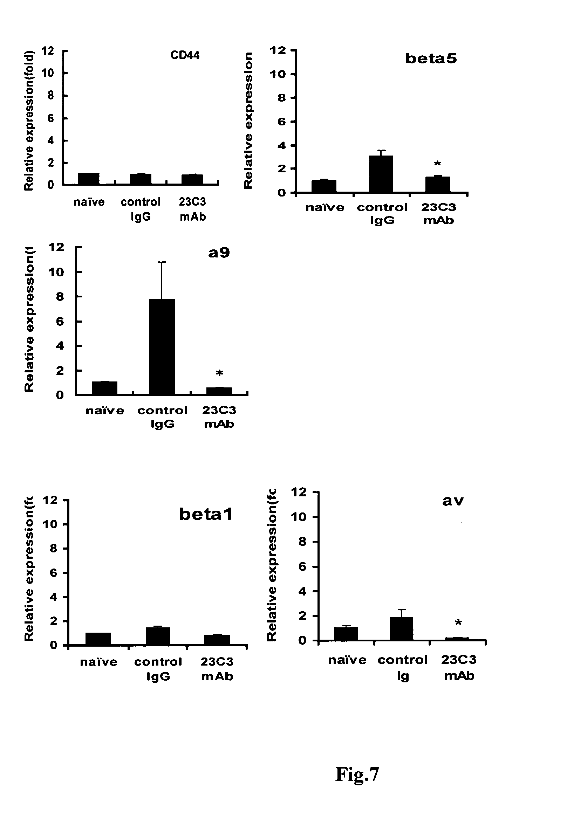

The experimental results were shown in Figure 7, wherein CD44, β1, and α8 in control group and treatment group were not significantly different from those in normal mice; whereas αv, β3, β5, α4, and α9 in control group were significantly higher than those in normal mice, and mRNA levels thereof were significantly reduced after 23C3 monoclonal antibody treatment. The results suggest that OPN receptors αv, β3, β5, α4, and α9 may be involved in the pathogenesis of arthritis; and 23C3 can regulate the mRNA levels of OPN receptors by neutralizing tissue OPN protein.

**Table 1 Primers of various OPN receptors**

| Specific primers for the molecules detected in Real-time PCR | | | |
|---|---|---|---|
| Name | SEQ ID NO: | primer | Sequence (5'→ 3') |
| GAPDH | 26 | FW | CTTCACCACCATGGAGAAGGC |
| | 27 | RV | GGCATGGACTGTGGTCATGAG |
| OPN | 28 | FW | GATTTGCTTTTGCCTGTTTGG |
| | 29 | RV | TGAGCTGCCAGAATCAGTCACT |
| αv | 30 | FW | CTCTGAAACCTGAGGTGCTTCT |
| | 31 | RV | GGGCGAAGTAAATGTAACCT |
| α4 | 32 | FW | GAATCCAAACCAGACCTGCGA |
| | 33 | RV | TGACGTAGCAAATGCCAGTGG |
| α8 | 34 | FW | TTGAAGCTGTCAGCTAGACC |
| | 35 | RV | CACACCACCATCCTGGTCAC |
| α9 | 36 | FW | TTAAGTGTCGTGTCCATACC |
| | 37 | RV | GATGTTCTTCCAACGATGGG |
| β1 | 38 | FW | CCTACTGGTCCCGACATCAT |
| | 39 | RV | GTCCCACTTGGCATTCATTT |
| β3 | 40 | FW | TGATATCCTGGTGGTACTGCTG |
| | 41 | RV | GTTGTTTGCTGTGTCCCACTTA |
| β5 | 42 | FW | CAACAAATCCTACAATGGCTCA |
| | 43 | RV | ACACCAGAGTCTCCTTGCTTTC |
| CD44 | 44 | FW | TGAAGTTGGCCCTGAGCAA |
| | 45 | RV | CTCGGAATTACCACATTTCCTTCT |
| IL-1β | 46 | FW | CAACCAACAAGTGATATTCTCCA |
| | 47 | RV | GATCCACACTCTCCAGCTGCA |
| IL-6 | 48 | FW | GAGGATACCACTCCCAACAGACC |
| | 49 | RV | AAGTGCATCATCGTTGTTCATACA |
| TNF-α | 50 | FW | CATCTTCTCAAAATTCGAGTGAC |
| | 51 | RV | TGGGAGTAGACAAGGTACAACC |

| | | | |
|---|---|---|---|
| FW= Forward; RV= Reverse | | | |

### Experiment I-6 Cytokines mRNA levels in CIA mice joint tissue of different treatment groups

At day 14, the joint tissues of CIA mice in different treatment groups of experiment I-1 were collected. The skin and muscle tissues were removed. Total RNA was extracted with RNeasy Mini Kit (QIAGEN) and subjected to reverse transcription with Sensiscript RT Kit (QIAGEN) for cDNA. Cytokine primers were designed according to the requirements of real-time PCR (as shown in Table 1). The tissue mRNA levels of cytokines were detected by real-time PCR using SYBR Green Master Mix (Applied Biosystems). The PCR procedure was the same as that used in experiment 4. The experimental data was analyzed using ABI PRISM 7900 sequencing system (Applied Biosystems).

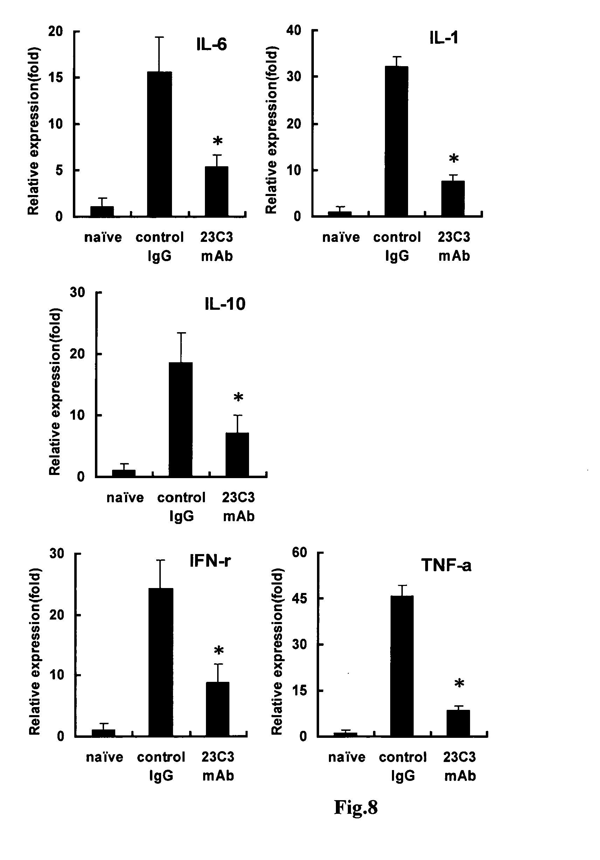

The experimental result was shown in Figure 8, wherein the mRNA levels of pro-inflammatory cytokines TNF-α, IL-1 and IL-6 in control group were significantly higher than those in normal mice, and the mRNA levels were significantly reduced after monoclonal antibody 23C3 treatment. Meanwhile, the mRNA levels of Th1-type cytokine IFN-γ and Th2-type cytokine IL-10 were significantly reduced after monoclonal antibody 23C3 treatment. The results suggest that 23C3 can reduce the level of pro-inflammatory cytokines thereby reducing the degree of inflammation of arthritis; and 23C3 can not only down-regulate Th1-type immune response, but also down-regulate Th2-type immune response.

### Experiment I-7. In vitro lymphocyte recall response changes in different treatment groups

The spleens and lymph nodes of CIA mice in different treatment groups of Experiment I-1 were aseptically separated, passed 100 mesh steel screen, washed with PBS for two times, and treated with lymphocyte separating medium to separate spleen lymphocyte. The resultant was centrifuged at 2000 rpm for 20 minutes. The lymphocyte layer was pipetted and washed with PBS for 2 times. The cells were counted, and the lymphocyte concentration was adjusted to 5 × 10⁶ cells/ml. 5 × 10⁵ lymphocytes were added to each well and were stimulated with different concentrations of chicken type II collagen. The plate was incubated at 37°C, 5% CO₂ for 72 hours. The supernatant collected at hour 56 was used for the detection of cytokine IFN-γ and IL-10. At the same time, 100ul culture medium containing ³H (0.5 µCi) was added into each well, and the plate was cultured for another 16 hours. Cells were then collected with cell collector and detected with γ liquid scintillation counter.

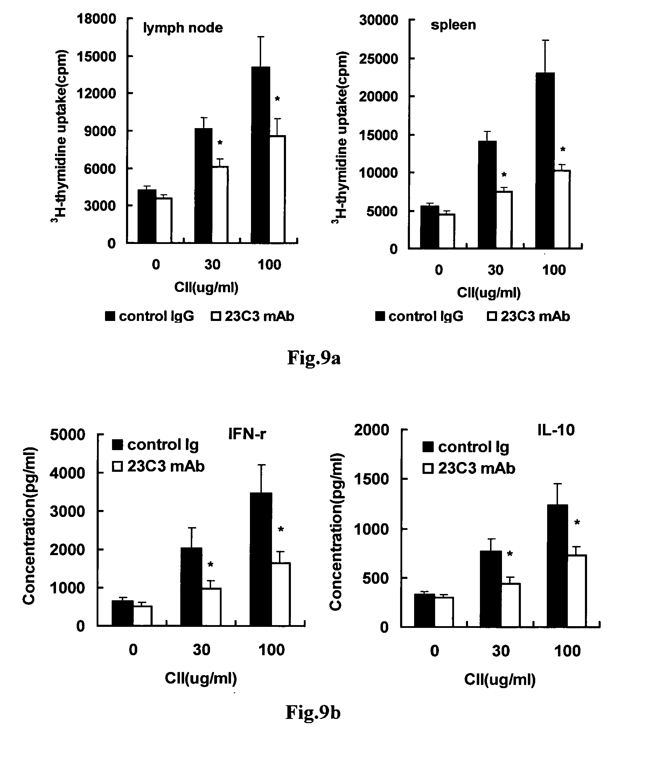

The experimental result was shown in Figure 9. The response ability to chicken type II collagen in 23C3 mAb treatment group was significantly lower than that in control group, and the levels of Th1-type cytokine IFN-γ and Th2-type cytokine IL-10 were significantly lower than those in control group during the response.

### Experiment I-8. The serum autoantibodies levels in CIA mice of different treatment groups

The DBA/1J mice serums of different time points (0, 7, 14 or 28 days) from different treatment groups (experiment I-1) were collected, and detected for the serum titer of anti-type II collagen autoantibodies IgG1, IgG2a and total Ig using ELISA method. Specifically, venous blood from mice outer canthus was collected, allowed to stand for 2 hours at room temperature, and centrifuged at 4000rpm for 10 minutes to separate the serum. 96-well plate was coated with 100ng/well chicken type II collagen, 4°C overnight, washed with PBS, blocked with 10% skimmed milk for 2 hours, and then washed with PBST for three times. The serum samples were serial diluted beginning with 1:2000, and diluted samples were added to the 96-well plate, incubated at room temperature for 1 hour. After the plate was washed with PBST for five times, HRP labeled rabbit anti-mouse IgG1, IgG2a and total Ig antibody were added into the plate. The plate was incubated for 2 hours at 37°C, washed for five times, and developed with substrate o-phenylenediamine. OD450 absorbance was detected. The autoantibody titer of autoantibody in serum sample was calculated according to the standard curve.

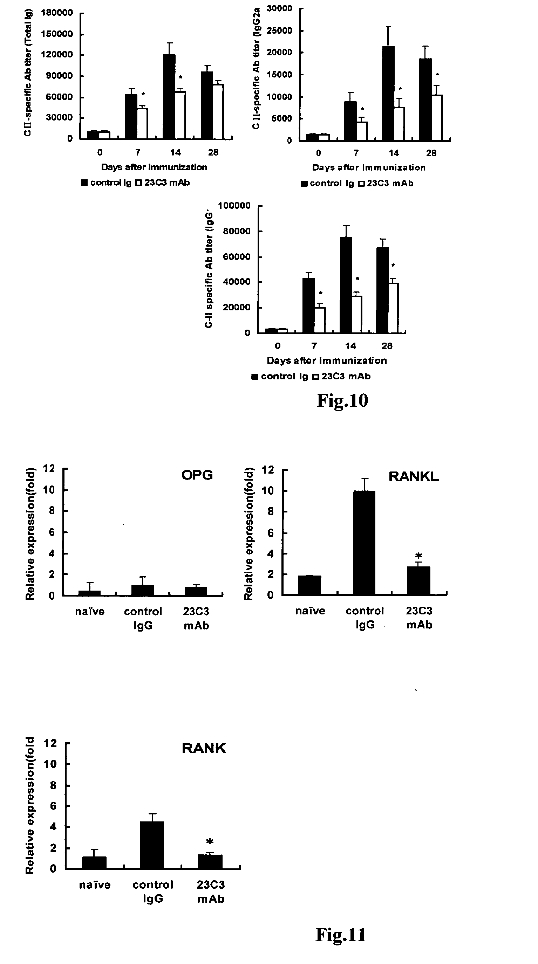

The result was shown in Figure 10, wherein the IgG1, IgG2a and total Ig serum titers in control group were significantly higher than those in the 23C3 treatment group, and reached the highest titers in day 14. The result suggested that autoantibody level could be correlated with the occurrence and severity of disease, and 23C3 monoclonal antibody could reduce the severity of arthritis by reducing IgG1, IgG2a, and total Ig levels.

### Experiment I-9. RANKL, RANK and OPG mRNA levels in the joint tissue of CIA mice in different treatment groups

At day 28, the joint tissues of mice in different treatment groups (experiment I-1) were collected. The skin and muscle tissues were removed. Total RNA was extracted with RNeasy Mini Kit (QIAGEN) and subjected to reverse transcription with Sensiscript RT Kit (QIAGEN) for cDNA. Primers for RANKL, RANK and OPG were designed according to the requirements of real-time PCR (as shown in Table 1). The tissue mRNA levels of RANKL, RANK and OPG were detected by real-time PCR using SYBR Green Master Mix (Applied Biosystems). The PCR procedure was the same as that used for human and mouse eukaryotic expression and purification step as indicated in the examples. The experimental data was analyzed using ABI PRISM 7900 sequencing system (Applied Biosystems).

The experimental result was shown in Figure 11, wherein the mRNA levels of regulatory factors RANKL and RANK, which promoted osteoclast differentiation and proliferation, in control group were significantly higher than those in normal mice, and the mRNA levels thereof were significantly reduced after monoclonal antibody 23C3 treatment. OPG level showed no significant change in control group and treatment group. The result suggests that 23C3 can down-regulate the level of RANKL and RANK, and thereby inhibiting the function of osteoclast, as well as reducing bone destruction and absorption in arthritis.

### Experiment I-10. The urine D-pyr level in CIA mice of different treatment groups

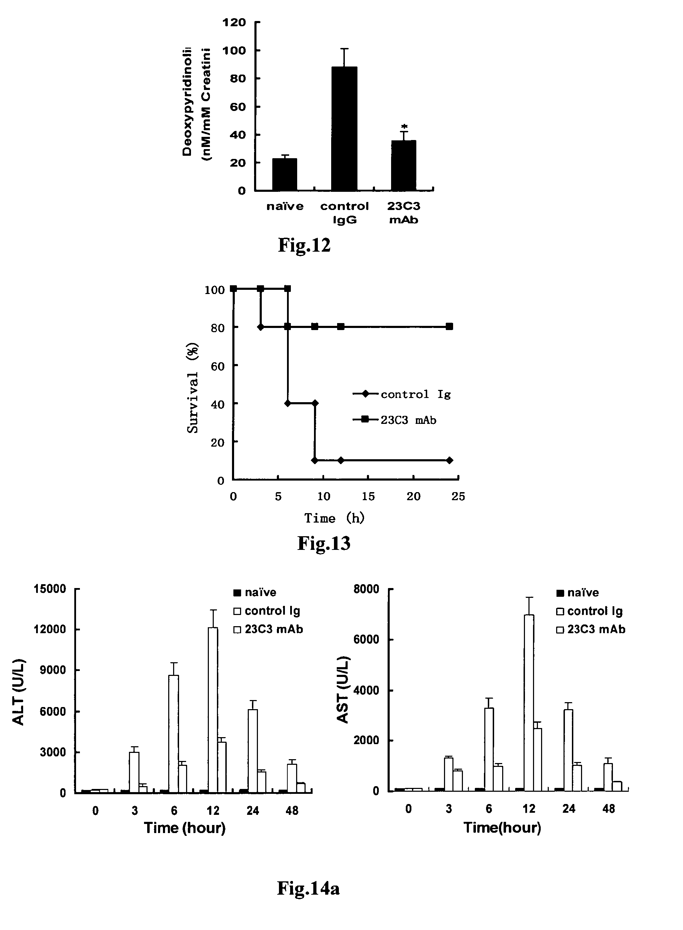

The urine of DBA/1J mice (experiment I-1) in different treatment groups was collected 30 days after the secondary immunization, and detected for D-pyr level using mice D-pyr ELISA kit (R & D). The result was shown in Figure 12, wherein the urine D-pyr level in 23C3 monoclonal antibody treatment group was significantly lower than that in control group.

### Experiment II. Effect of 23C3 monoclonal antibody on hepatitis model mice

### Experiment II-1. The effect of 23C3 monoclonal antibody on the mortality of hepatitis model mice

C57BL/6 (male, 8-10 weeks old) mice (purchased from Experimental Animal Center of the Second Military Medical University) were administrated with irrelevant control antibody Ig (Sigma) or 23C3 monoclonal antibody (200 µg/mouse, and 10 mice for each group), via intraperitoneal injection. After 2 hours, 200 µl ConA (30 mg/ml) was injected to the tail veins of the mice to induce acute severe hepatitis. The effect of 23C3 monoclonal antibody on mortality in hepatitis mice was continually observed for 24 hours.

The experimental result was shown in Figure 13. 23C3 mAb could significantly reduce the mortality as compared to control group, wherein the protective effect of monoclonal antibody 23C3 was stronger.

### Experiment II-2. The liver protective effect of 23C3 monoclonal antibody on hepatitis model mice

C57BL/6 (male, 8-10 weeks old) mice were administrated with irrelevant control antibody Ig (Sigma) or 23C3 monoclonal antibody (200 µg/mouse, and 10 mice for each group), via intraperitoneal injection. After 2 hours, 200 µl ConA (30 mg/ml) was injected to the tail vein of to the mice to induce acute severe hepatitis. 0, 3, 6, 12, 24 and 48 hours after injection of ConA, the mice serum of each treatment group was collected and detected for ALT and AST levels. The mouse livers of each group were taken at 12 hour, stained by HE and TUNEL for the observations of liver necrosis, inflammatory infiltration and apoptosis.

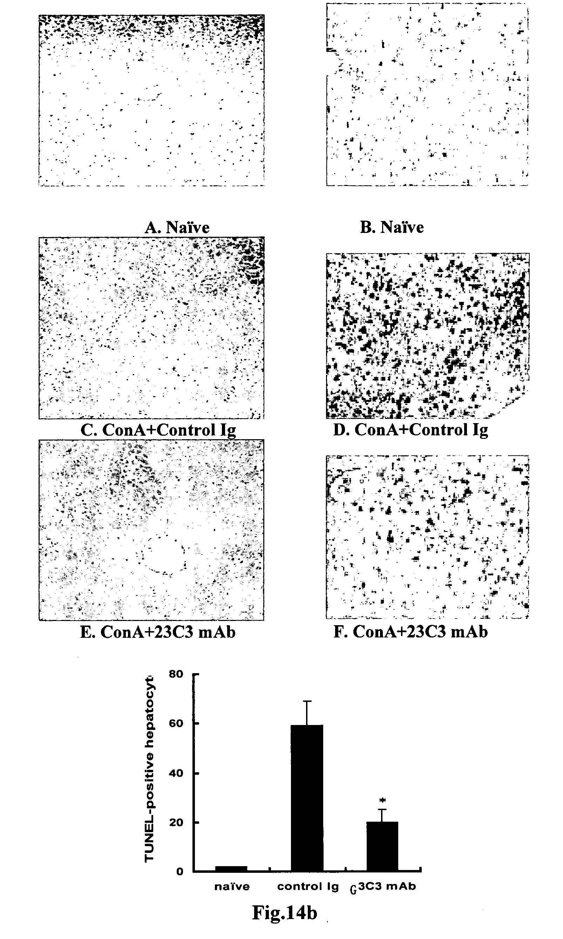

The experimental result was shown in Figure 14. As shown in Figure 14a, the serum ALT and AST levels in 23C3 monoclonal antibody treatment group were significantly reduced as compared to those of control group, wherein the ALT and AST levels in 23C3 monoclonal antibody treatment group were always lower than other groups at any time points. As shown in Figure 14b, 23C3 monoclonal antibody group had a low liver necrosis extent, and the inflammatory infiltration level was significantly lower than that of control group. As shown in TUNEL staining, liver cell apoptosis was serious in control group, with an apoptosis percentage of nearly 60%, while the percent apoptosis in 23C3 treatment group was about 20%. Therefore, the above liver injury indicator and pathological indicator both suggest that 23C3 has obvious protective effect on liver injury.

### Experiment II-3. The OPN level in the plasma and liver tissue of hepatitis model mice in different treatment groups

The C57BL/6 mice plasma of different time points (0, 3, 6, 12 or 24 hours) in different treatment groups (experiment II-2) was collected, and detected for OPN protein concentration using mouse OPN ELISA kit. Specifically, venous blood from mice outer canthus was collected, and centrifuged immediately at 4000 rpm for 10 minutes to separate the plasma. The 1:200 dilution was added into 96 well plate of ELISA detection kit, incubated at room temperature for 1 hour, and washed with PBST for five times. Biotin labeled anti-OPN primary antibody was then added into the plate, and the plate was incubated at room temperature for 1 hour and washed for five times. Then, avidin labeled secondary antibody was added into the plate. The plate was incubated at room temperature for 1 hour, washed for six times, and developed with TMB substrate. OD450 absorbance was detected. The plasma OPN concentration was calculated according to the standard curve.

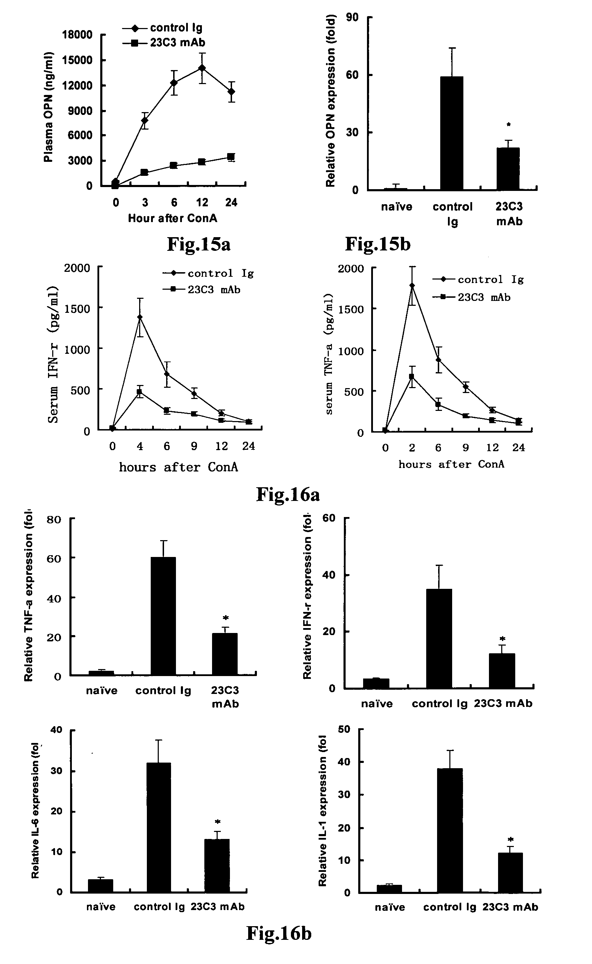

The result was shown in Figure 15a. The OPN level in control group was significantly higher than that in 23C3 treatment group, and the OPN level in 23C3 treatment group was always the lowest at any time point.

At hour 6, the liver tissue of different treatment groups (experiment II-2) was collected. The total RNA was extracted from the tissue with RNeasy Mini Kit (QIAGEN) and subjected to reverse transcription using Sensiscript RT Kit (QIAGEN) for cDNA. OPN primers were designed according to the requirements of real-time PCR (as shown in Table 1). OPN mRNA level was detected by real-time PCR using SYBR Green Master Mix (Applied Biosystems). The PCR procedure was: 50°C for 2 mins; 95°C for 10 mins; 40 s of 95°C for 15 sec and 60°C for 60 sec. The internal standard was GAPDH gene. The experimental data was analyzed using ABI PRISM 7900 sequencing system (Applied Biosystems).

The experimental result was shown in Figure 15b, wherein the liver tissue OPN level in 23C3 mAb treatment group was much lower than the OPN level in control group. The result suggests that OPN monoclonal antibody can regulate OPN mRNA level by neutralizing OPN protein in the tissue.

### Experiment II-4. The cytokines levels in the plasma and liver tissue of hepatitis model mice in different treatment groups

The C57BL/6 mice plasma of different time points (0, 3, 6, 12 or 24 hours) in different treatment groups (experiment II-2) were selected, the TNF-α and IFN-γ protein concentrations were detected using mouse TNF-α, IFN-γ ELISA kit (R&D). Specifically, venous blood from mice outer canthus was collected, allowed to stand at room temperature for 2 hours and separated for the serum.

The 1:100 dilution was added into 96 well plate of ELISA detection kit, incubated at room temperature for 1 hour and washed with PBST for five times. Biotin labeled anti-TNF-α or IFN-γ primary antibod was then added into the plate, and the plate was incubated at room temperature for 1 hour and washed for five times. Avidin labeled secondary antibody was then added into the plate. The plate was incubated at room temperature for 1 hour, washed for six times, and developed with TMB substrate. OD450 absorbance was detected. The plasma TNF-α and IFN-γ concentrations were calculated according to the standard curve.

The result was shown in Figure 16a. The cytokine TNF-α and IFN-γ levels in control group were significantly higher than those in 23C3 treatment group.

At hour 6, the liver tissues of different treatment groups were collected. The total RNA in the tissue was extracted with RNeasy Mini Kit (QIAGEN) and subjected to reverse transcription using Sensiscript RT Kit (QIAGEN) for cDNA. Primers for cytokines were designed according to the requirements of real-time PCR (as shown in Table 1). Cytokine mRNA level was detected by real-time PCR using SYBR Green Master Mix (Applied Biosystems). The PCR procedure was the same as that used in example 3. The experimental data was analyzed using ABI PRISM 7900 sequencing system (Applied Biosystems).

The experimental result was shown in Figure 16b, wherein the pro-inflammatory cytokines TNF-α, IL-1β and IL-6 mRNA levels in control group were significantly higher than those in normal mouse, and the mRNA levels thereof were significantly reduced after monoclonal antibody 23C3 treatment. Meanwhile, the mRNA level of Th1-type cytokine IFN-γ was significantly reduced in monoclonal antibody 23C3 treatment group. The result suggests that 23C3 can reduce the level of pro-inflammatory cytokine, and thereby reducing the degree of inflammation of liver. Moreover, 23C3 could also down-regulate Th1-type immune response.

### Experiment III. The effect of 23C3 mAb on Crohn's disease model mice

### Experiment III-1. The effect of 23C3 monoclonal antibody on body weight, disease activity index (DAI) and spleen index in Crohn's disease model mice

Dextran sulfate sodium (DSS) was used to induce experimental colitis in 30 mice. That is, 30g/L DSS was given to the mice instead of drinking water for 7 days. The above mice were randomly divided into 3 groups: control, Ig control treatment group and 23C3 monoclonal antibody treatment group. Intraperitoneal administration began at the modeling day and lasted for 3 consecutive days, 200ug/mouse/time. The body weights of the experimental mice were recorded every day. The disease activity index was scored every day according to stool character, occult blood test and the change of body weight (scoring criteria was shown in table 2);

**Table 2. Disease activity index (DAI) scoring criteria**

| Score | Decrease of Body weight (%) | Stool character | Hematochezia |
|---|---|---|---|
| 0 | none | Normal | Negative |
| 1 | 1-5 | Normal | Negative |
| 2 | 6-10 | Semi-loose stools | Occult blood(+) |
| 3 | 10-15 | Semi-loose stools | Occult blood(+) |
| 4 | >15 | Loose stools | Visible Hematochezia |

| | | | |
|---|---|---|---|
| DAI = (body weight decrease score + stool character score + hematochezia score)/3 | | | |

At day 7, the mouse spleens were taken and weighed to calculate the spleen index (spleen weight/body weight).

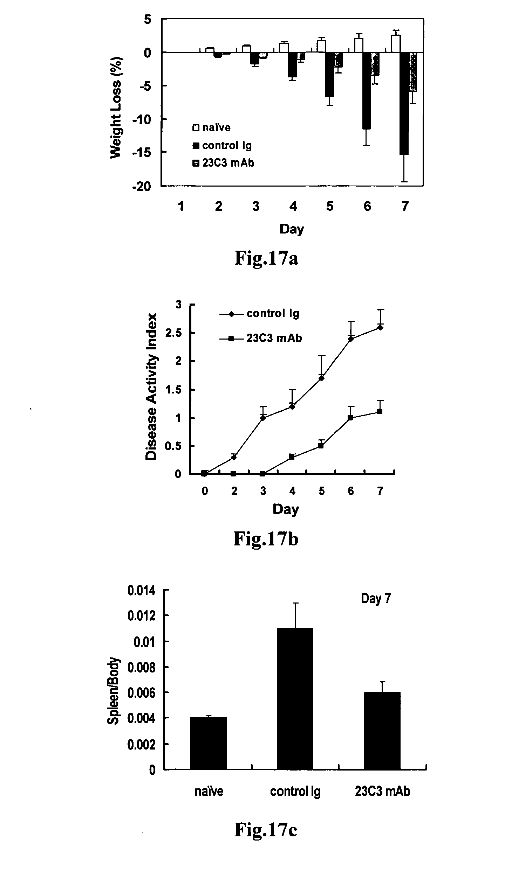

The experimental results were shown in Figure 17a, 17b, 17c. Loose stool or diarrhea, hematochezia and weight loss were observed in all the animals in control treatment group, whereas in mAb treatment group, the stools were relatively shaped, bloody stool and weight loss were not evident. As compared to control treatment group, the disease activity index in mAb treatment group was significantly reduced. The spleen index in control treatment group was significantly higher than that in normal mice, while the increase in the spleen index was not obvious in mAb treatment group.

### Experiment III-2. The histopathologic changes of colon in Crohn's disease model mice of different treatment groups

At day 7, the colon tissue with pathologic changes of different treatment groups (experiment III-1) were taken, washed with PBS, fixed in 10% formalin, paraffin-embedded, sliced continuously into 2 µm sections, and stained with HE. The histological changes in different treatment groups were observed using light microscope. The specific scoring criteria were shown in Table 3.

**Table 3. Histopathologic scoring criteria**

| Scor e | Inflammation degree | Inflammation scope | Ulcer depth | Ulcer Range |
|---|---|---|---|---|
| 0 | None | None | None | 1-25% |
| 1 | Minimal | Mucosa | Basal 1/3 | 26-50% |
| 2 | Moderate | Mucosa and submucosa | Basal 2/3 | 51-75% |
| 3 | Serious | Total | Only integrated in the surface epithelium | 76-100% |
| 4 | | | Full-thickness ulcer | |

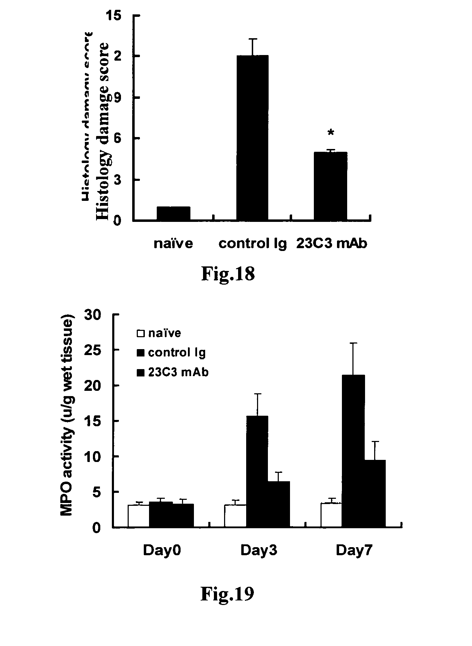

The result was shown in Figure 18. It was observed under light microscope that in the control treatment group, the colon histological structure was incomplete, the ulcer was deep and the ulcer range was large with inflammatory exudation, a large number of lymphocytes and neutrophils were infiltrated, and mononuclear cells and eosinophils varied in amount were observed; as compared with control group, in 23C3 monoclonal antibody treatment group, the histological structure was complete, inflammation and ulcer extent was minor, lymphocytes and neutrophils infiltration was few, and the histopathologic score was significantly lower than that of the control treatment group.

### Experiment III-3. Effect of 23C3 monoclonal antibody on intestinal tissue myeloperoxidase (MPO) activity in Crohn's disease model mice

Intestinal tissue myeloperoxidase (MPO) activity was detected using commercial reagent for detection. At day 0, 3 and 7, the colon tissues of each treatment group (experiment III-1) were taken, weighted the same weight, shredded, and homogenized for 30 sec in ice bath (in 0.5% HTAB containing buffer). The homogenate was sonicated for 10 sec, repeated freeze-thaw for three times, centrifuged at 15000g for 15 mins. 0.1 ml homogenate supernatant was added to 2.9ml of 0.167mg/ml O-dianisidine hydrochloride, and then 0.0005% H₂O₂ was added. OD460 was detected. The MPO activity was calculated as follow, one MPO activity unit equaled to 1 µmol peroxidase degradation in 1 minute.

The result was shown in Figure 19. MPO activity of each group was time-dependent, with the progress of inflammation, MPO activity increased gradually. At day 3 and 7, the MPO levels in control treatment group were significantly higher than those of 23C3 monoclonal antibody treatment group, and 23C3 group had the lowest MPO activity levels.

### Experiment III-4. The intestinal tissue TNF-α, IL-1β and IL-6 mRNA levels in Crohn's disease model mice of different treatment groups

At day 3 and 7, the mice intestinal tissues of different treatment groups (experiment III-1) were taken. Total RNA was extracted using RNeasy Mini Kit (QIAGEN) and subjected to reverse transcription with Sensiscript RT Kit (QIAGEN) for cDNA. Primers for various cytokines were designed according to the requirements of real-time PCR (as shown in Table 1). Cytokine mRNA levels were detected by real-time PCR using SYBR Green Master Mix (Applied Biosystems). PCR procedure was the same as that used in Example 3. The experimental data was analyzed using ABI PRISM 7900 sequencing system (Applied Biosystems).

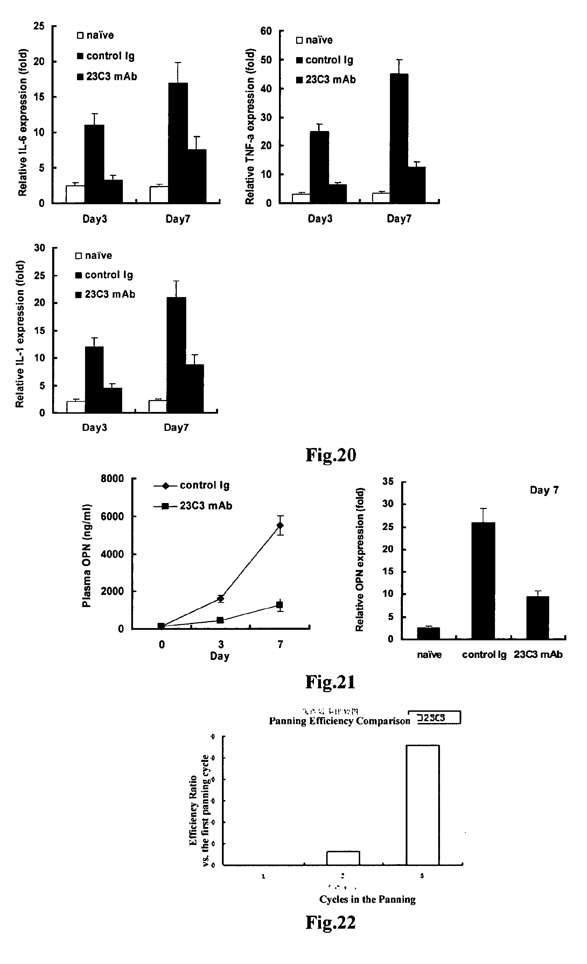

The experimental result was shown in Figure 20, wherein the mRNA levels of pro-inflammatory cytokines TNF-α, IL-1β and IL-6 were significantly higher in control group than in normal mice, and the mRNA levels significantly reduced after 23C3 treatment. The result suggests that 23C3 can reduce the degree of colon inflammation by reducing the levels of pro-inflammatory cytokines.

### Example III-5. The intestinal tissue and plasma OPN levels in Crohn's disease model mice of different treatment groups

The C57BL/6 mice plasma of different treatment groups (experiment III-1) were collected at different time points (day 0, 3 and 7), and detected for OPN protein concentration using mouse OPN ELISA kit. Specifically, venous blood from mice outer canthus was collected, and centrifuged immediately at 4000 rpm for 10 minutes to separate the plasma. The 1:200 dilution was added into 96 well plate of ELISA detection kit, incubated at room temperature for 1 hour, and washed with PBST for five times. Biotin labeled anti-OPN primary antibody was then added into the plate, and the plate was incubated at room temperature for 1 hour. After the plate was washed for five times, avidin labeled secondary antibody was added. Then, the plate was incubated at room temperature for 1 hour, washed for six times, and developed with TMB substrate. OD450 absorbance was detected. The plasma OPN concentration was calculated according to the standard curve.

The result was shown in the left panel of Figure 21. The OPN level in control group was significantly higher than that in 23C3 treatment group, and was always higher than that in 23C3 treatment group at any time.

At day 7, the colon tissues of different treatment groups (experiment III-1) were taken. The total RNA was extracted with RNeasy Mini Kit (QIAGEN) and subjected to reverse transcription using Sensiscript RT Kit (QIAGEN) for cDNA. OPN primers were designed according to the requirements of real-time PCR (as shown in Table 1). OPN mRNA level was detected by real-time PCR using SYBR Green Master Mix (Applied Biosystems). The PCR procedure was: 50°C for 2 mins; 95°C for 10 mins; 40 cycles of 95°C for 15 sec and 60°C for 60 sec. The internal standard was GAPDH gene. The experimental data was analyzed using ABI PRISM 7900 sequencing system (Applied Biosystems).

The experimental result was shown in the right panel of Figure 21, wherein the colon tissue OPN level in 23C3 mAb treatment group was significantly lower than the OPN level in control group. The result suggests that OPN monoclonal antibody can regulate OPN mRNA level by neutralizing OPN protein in the tissue.

### Experiment IV. Effect of c23C3 and h23C3 on in vitro lymphocyte migration, immune response and survival

### Experiment IV-1. The inhibitory effect of c23C3 and h23C3 antibodies on lymphocyte migration

CIA mice spleens were aseptically separated, passed 100 mesh steel screen, washed with PBS for two times, treated with lymphocyte separating medium to separate spleen lymphocyte, and centrifuged at 2000 rpm for 20 minutes. The lymphocyte layer was pipetted and washed with PBS for 2 times. The lymphocytes were counted, and adjusted to a concentration of 5 × 10⁶ cells/ml. 24-well tissue culture plate having a 5µm filter membrane at the upper layer was pre-coated with 10ug OPN at the lower layer. 5 × 10⁵ lymphocyte as well as the following antibodies were added into each well in the upper layer: 23C3, Her-2 antibody (irrelevant antibody control), c23C3 and h23C3. The plate was incubated at 37°C, 5% CO₂ for 2 hours, washed with PBS twice. The number of cell in the lower layer was calculated by taking the average of counted numbers in five high power fields and then multiplied by 50.

The experimental result was shown in Figure 32. Both c23C3 and h23C3 could effectively block the migration of lymphocytes in a dose dependent manner, but the inhibitory effect was a slightly weaker than murine origin 23C3 mAb.

### Experiment IV-2. The inhibitory effect of c23C3 and h23C3 antibodies on lymphocyte immune response

CIA mice spleens were aseptically separated, passed 100 mesh steel screen, washed with PBS for two times, treated with lymphocyte separating medium to separate spleen lymphocyte, and centrifuged at 2000 rpm for 20 minutes. The lymphocyte layer was pipetted and washed with PBS for 2 times. The lymphocytes were counted, and adjusted to a concentration of 5 × 10⁶ cells/ml. 5 × 10⁵ lymphocytes as well as 30 µg/ml of chicken type II collagen and different concentrations of 23C3, control Ig, c23C3 and h23C3 were added into each well. The plate was incubated at 37°C, 5% CO₂ for 72 hours. 100ul culture medium containing ³H (0.5 µCi) was added into each well, and the plate was cultured for another 16 hours. Cells were then collected with cell collector and detected with γ liquid scintillation counter.

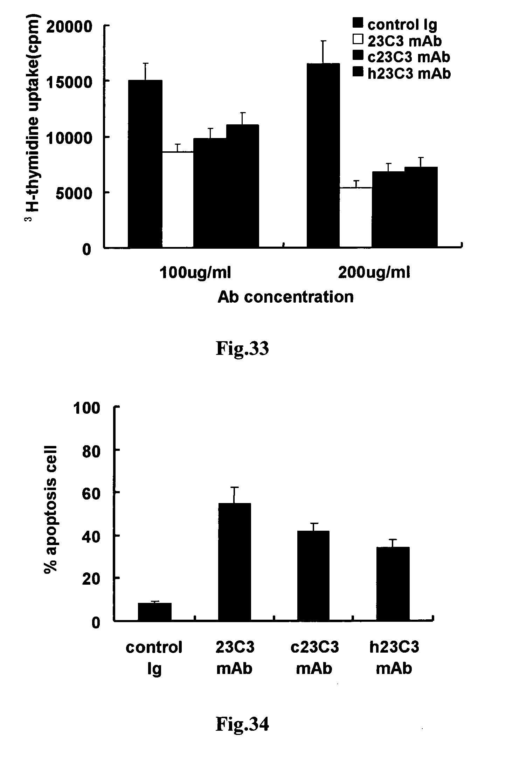

The experimental result was shown in Figure 33. Both c23C3 and h23C3 could effectively inhibit the lymphocyte immune response to chicken type II collagen in a dose dependent manner, but the inhibitory effect was slightly weaker than murine origin 23C3 mAb.

### Experiment IV-3. Effect of c23C3 and h23C3 antibodies on the survival of activated lymphocyte

CIA mice spleens were aseptically separated, passed 100 mesh steel screen, washed with PBS for two times, treated with lymphocyte separating medium to separate spleen lymphocytes, and centrifuged at 2000 rpm for 20 minutes. The lymphocyte layer was pipetted and washed with PBS for 2 times. The lymphocytes were counted, and adjusted to a concentration of 5 × 10⁶ cells/ml. 5 × 10⁵ lymphocyte as well as 100µg/ml of chicken type II collagen and different concentrations of 23C3, control Ig, c23C3 and h23C3 were added into each well. The plate was incubated at 37°C, 5% CO₂ for 72 hours. The cells were collected, to which Annexin V-FITC was added. Cell apoptosis was detected through flow cytometry.

The result was shown in Figure 34, wherein c23C3 and h23C3 antibodies could effectively promote the apoptosis of activated lymphocytes, and the proportion of apoptotic cells were 42% and 34% respectively. The murine region 23C3 monoclonal antibody induced the apoptosis of activated lymphocytes at the ratio of 55%, which was higher than those of chimeric and humanized anti-OPN antibodies.

### Experiment V. Identification of the epitope of the anti-hOPN (23C3) monoclonal antibody

### Experiment V-1. Panning of the epitope of anti-hOPN (23C3) monoclonal antibody

The experiment was performed using (Phage) random peptide library panning method, and the whole process was carried out in a 96-well plate. The plate was coated with 100ug/ml 23C3 monoclonal antibody (100 µl/well) at 4°C overnight, blocked with 10% skimmed milk (diluted with TBST) overnight, and washed with 1 × TBST (Tween-20, 0.1%) for six times. To the plate were added the phage random peptide library (purchased from NEB, Ph.D.-12™ Phage Display Peptide Library Kit) 4×10¹⁰ pfu and 100 µl normal mouse serum, and gently agitated at room temperature for 1 hour. The plate was washed with 1 × TBST (Tween-20, 0.1%), 1 × PBS (pH5.0), and 1 × PBS (pH3.5) (each for five times), and then eluted with glycine-Cl (containing 1mg/ml BSA, pH 2.2) with gently agitated at room temperature for 15 minutes before neutralizing with 15ul Tris-Cl (pH 9.1). 10 µl resultant was taken out for titer, and the rest was used for amplification. Amplification products were precipitated by PEG/NaCl, and the titer was measured, on which the second round of panning was performed. The third round of panning was performed with the same process. Each time the same number of phages (4 × 10¹⁰ pfu) was added, and the number of the output phage after three rounds of panning was 2.76 × 10³ pfu, 3.45 × 10⁵ pfu and 3.08 × 10⁶ pfu, respectively. The panning efficiency increased at a 125-fold and 1116-fold relative to that of the first round, respectively. The result suggests that the panning enrichment effect is significant (as shown in Figure 22).

### Experiment V-2. Phage clone ELISA and Western blot assay

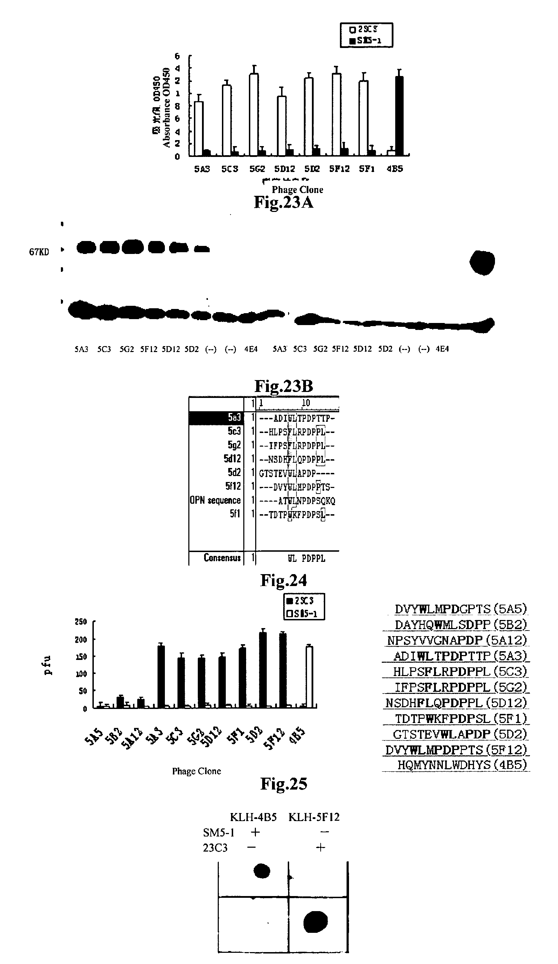

ELISA was carried on a 96-well plate. Each well was coated with 50 µl of 100 µg/ml 23C3 monoclonal antibody at 4°C overnight, blocked with 10% skimmed milk (TBST diluted) at 37°C for 2 hours, washed with 1 × TBST (Tween-20, 0.1%) for five times. The supernatant of each monoclonal phage amplification product was diluted with 1 × TBS to 5 × 10⁸ pfu/50µl. The control antibody was mouse monoclonal antibody SM5-1 (prepared by the Cancer Institute of the Second Military Medical University, and according to the procedure disclosed in *"*Arch Dermatol Res. Dec. 2000; 292 (12): 583-9, the same method was used hereinafter.). The negative control phage was 4E4 (SM5-1 positive clone, ibid. The same negative control phage was used hereinafter). Binding was carried out at room temperature for 1 hour. The resultant was washed with 1 × TBST (Tween-20, 0.1 %) for five times, and then 200 µl of 1:5000 diluted HRP labeled anti-M13 antibody (Pharmacia # 27-9411-01) was added into each well. The plate was shaken at room temperature for 1 hour and washed with 1 × TBST (Tween-20, 0.1%) for five times. Fresh substrate prepared by mixing reagent A and reagent B of ELISA detection kit (Jinmei, A:B = 1:1, 50ul/well) was added into each well, and the plate was incubated at room temperature for 1-5 minutes before adding 2N H₂SO₄ to terminate the reaction. The reaction of each clone to 23C3 and control antibody SM5-1 was detected in triplicate. OD450 data showed that the response of the positive clones to the antibody was specific (as shown in Figure 23A).

Western blot process was as follows: the supernatant of the amplified phage monoclonal was purified by 20% PEG/NaCl precipitation, then subject to 10% SDS-PAGE electrophoresis (1 × 10¹⁰pfu/lane) under 360mA constant current for 1 hour to transfer to a nitrocellulose membrane. The membrane was blocked with 10% skim milk at 4°C overnight or at room temperature for two hours. After being washed with 1 × TB ST (Tween-20, 0.1%) for three times (10 minutes/ time), the membrane was reacted with 10 µg/ml primary antibody at room temperature for 1 hour, and washed with 1 × TBST (Tween-20, 0.1%) for five times (10 minutes/ time). The membrane was then reacted with 1:1000 diluted HRP labeled rabbit anti-mouse IgG (Beijing Zhongshan Company) at room temperature for 1 hour, treated with ECL kit (Tiangen Company) for 1-2 minutes, and then pressed and exposed with medical blue-sensitive X-ray film. The result was shown in Figure 23B. The left panel of Figure 23B was the hybridization result of antibody 23C3, and the right panel was the hybridization result of irrelevant antibody SM5-1. These results indicated that the response between the positive clone and antibody was specific (control antibody was mouse monoclonal antibody SM5-1, negative control phage was 4E4 (SM5-1 positive clone)).

### Experiment V-3. Sequencing and sequence analysis

Single-stranded DNA Extraction Kit (Shanghai Jierui Company) was used to prepare the template. The sequencing was performed using -96 primer, and the sequence was read with Chromas. Among 25 positive clones were panned out 15 independent sequences (analyzed by AlignX). The result was shown in Figure 24, which indicates the possible epitope of 23C3 was WLXPDP.

### Experiment V-4. The binding ability of phage clone to antibody

Each clone obtained in experiment V-3 was added at 5 × 10⁷ pfu into an antibody coated 96 well plate, and was panned with the same conditions. (Control antibody: SM5-1; irrelevant control phage: 4E4). The titers of the phages were detected after elution (referring to *Blood* 2006-04-014639).

The results showed that: the epitope in hOPN sequence was W43LNP46DP48, wherein W43, P46, and P48 played an important role in mediating 23C3-hOPN binding. Moreover, the clones with the highest binding ability were: 5F12: DVYWLMPDPPTS synthetic short peptide, and 5D2:GTSTEVWLAPDP synthetic short peptide. The result was shown in Figure 25.

### Experiment V-5. The specific binding assay of synthetic short peptides and antibody

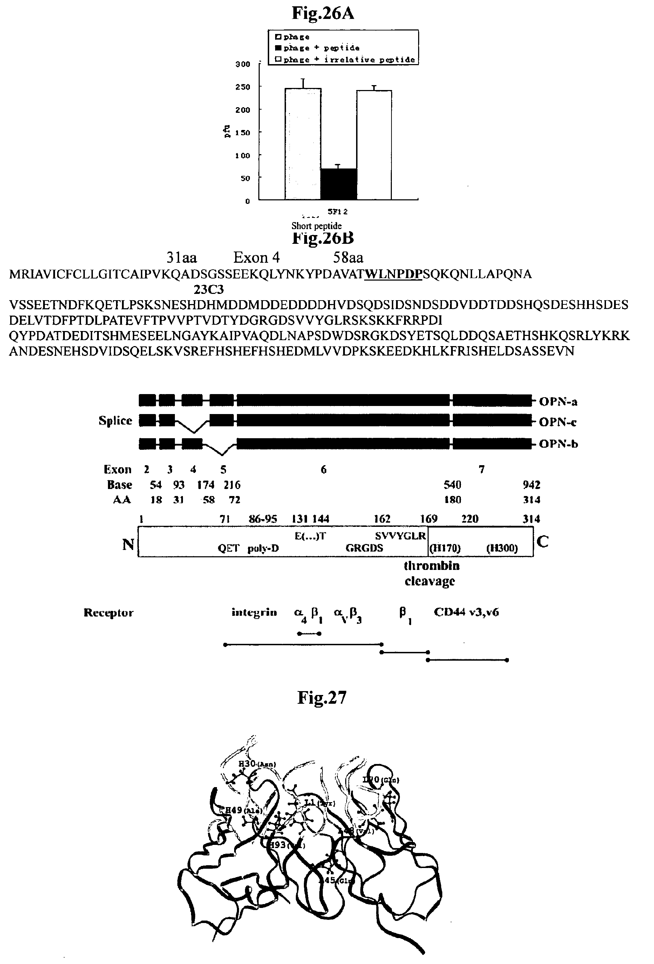

Dot-blot assay was as follows: three dots of 3x10ng KLH-4B5 (irrelevant control) and KLH-5F12 respectively, were pointed. Each short peptide obtained in experiment V-4 was used to hybrid with three different antibodies. After being blocked and washed, the membrane was reacted with primary antibody at room temperature for 1 hour. Then, the membrane was washed again, and reacted with HRP-labeled anti-mouse secondary antibody at room temperature for 1 hour. After being washed, the membrane was pressed and exposed. Irrelevant short peptide did not react with 23C3, while KLH-5F12 specifically reacted with the corresponding antibody. The result was shown in Figure 26A.

Blocking assay of short peptide on phage-antibody binding was as follows: 96 well plate was coated with 100ug/ml antibody (50 µl/well) at 4°C overnight, blocked with 10% skimmed milk, and washed with 1 × PBST for three times until use. 100 µg/ml short peptide was mixed with phage until homogenous, and the homogenous solution was then added to the antibody-coated plate. The plate was gently shook at room temperature for 1 hour before washed with 1 × TBST (Tween-20, 0.1%), 1 × PBS (pH5.0), and 1 × PBS (pH3.5) (each for 5 times). After being eluted with glycine-Cl (containing 1mg/ml BSA, pH 2.2) for 15 minutes, the plate was neutralized with Tris-Cl (pH 9.1). Then, the titer was measured. Each peptide was divided into three groups: (1) phage; (2) plage + peptide; and (3) phage + irrelevant peptide. The blocking efficiency of the short peptide 5F12 having such a concentration was 71 %. The result was shown in Figure 26B.

The results showed that, the sequence of the epitope of 23C3 antibody was WLNPDP, which was located in the fourth exon of OPN. The location and sequence of the epitope in OPN protein were shown in Figure 27. The sequence was as set forth in SEQ ID NO: 11.

All documents mentioned in this specification are herein incorporated by reference to the same extent as if each individual document was specifically and individually indicated to be incorporated by reference. Further, it is understood that, after reading this specification, those skilled in the art can make variations and modifications to the present invention, and the scope of protection of the invention is determined in particular by the following claims.

## Claims

1. A functional epitope of osteopontin, wherein the functional epitope is WLXPDP, and wherein X is any amino acid.

2. An anti-osteopontin monoclonal antibody specifically binding to the functional epitope of claim 1.

3. The monoclonal antibody of claim 2, wherein the amino acid sequence of the CDR in the heavy chain variable region of the monoclonal antibody is selected from the group consisting of: IYAMD, RIRSQSNNYTTYYADSVKD and QMGDY; and the amino acid sequence of the CDR in the light chain variable region is selected from the group consisting of: RASENIYSFLA, AATNLAD and QHFWGTPFT.

4. The monoclonal antibody of claim 2, wherein the amino acid sequence of the heavy chain variable region in the monoclonal antibody is SEQ ID NO: 4 or SEQ ID NO: 8, and the amino acid sequence of the light chain variable region in the monoclonal antibody is SEQ ID NO: 6 or SEQ ID NO: 10.

5. The monoclonal antibody of claim 2, wherein the constant region of the monoclonal antibody is a mouse antibody constant region or a human antibody constant region.

6. A DNA molecule encoding the monoclonal antibody of any of claims 2-5.

7. A vector containing the DNA molecule of claim 6.

8. A host cell containing the vector of claim 7, or having the DNA molecule of claim 6 integrated in its genome.

9. An immunoconjugate containing:
(a) the monoclonal antibody of claim 2; and
(b) a conjugate moiety selected from the group consisting of a drug, a toxin, a cytokine, a radionuclide, and an enzyme.

10. A use of the anti-osteopontin monoclonal antibody of claim 2 or the immunoconjugate of claim 9 in the manufacture of a medicament for treating autoimmune disease.

11. A pharmaceutical composition comprising: the anti-osteopontin monoclonal antibody of claim 2 or the immunoconjugate of claim 9; and pharmaceutically acceptable carriers.

12. A kit for the detection of osteopontin containing the anti-osteopontin monoclonal antibody of claim 2 or the immunoconjugate of claim 9.

13. A method for the detecting the presence of or determining the content of osteopontin in a biological sample, which includes the following steps:
(i) contacting the sample with the anti-osteopontin monoclonal antibody of claim 2 or the immunoconjugate of claim 9;
(ii) detecting the formation of antigen-antibody complex,
wherein the formation of antigen-antibody complex suggests the presence of osteopontin in the sample, or wherein the quantitatively determined content of the antigen-antibody complex reflects the content of osteopontin in the sample.
